# EUROPEAN PATENT SPECIFICATION

(11) **EP 2 819 618 B1**
(45) Date of publication and mention of the grant of the patent: **02.05.2018**
(21) Application number: 13717984.2
(22) Date of filing: 28.02.2013
(51) Int. Cl.: A61F 2/24

(54) **SINGLE-RING CARDIAC VALVE SUPPORT**
MONOZYKLISCHER HERZKLAPPENTRÄGER
SUPPORT DE VALVULE CARDIAQUE À ANNEAU UNIQUE

(30) Priority: 28.02.2012 US 201261604083 P; 05.04.2012 US 201261620679 P; 29.04.2012 US 201261639924 P; 23.05.2012 US 201261650559 P; 16.01.2013 US 201361752996 P; 16.01.2013 US 201361752994 P
(43) Date of publication of application: 07.01.2015
(73) Proprietor: Mvalve Technologies Ltd., 4672211 Herzliya (IL)
(72) Inventor: BUCHBINDER, Maurice, La Jolla, California 92037 (US); DUBI, Shay, 69869 Tel-Aviv (US); TUBISHEVITZ, Amit, 6930079 Tel-Aviv (IL); EFTEL, Avi, 6407606 Tel-Aviv (IL)
(74) Representative: Turner, Craig Robert
(86) International application number: PCT/IL2013/000025
(87) International publication number: WO 2013/128436

(56) References cited:
- WO-A2-2012/031141
- US-A1- 2009 177 277
- US-A1- 2011 319 990

## Description

### BACKGROUND OF THE INVENTION

Heart valve regurgitation occurs when the heart leaflets do not completely close when the heart contracts. When the heart contracts, blood flows back through the improperly closed leaflets. For example, mitral valve regurgitation occurs when blood flows back through the mitral valve and into the left atrium when the ventricle contracts.

In some instances regurgitation occurs due to disease of the valve leaflets (e.g., primary, or "organic" regurgitation). Regurgitation can also be caused by dilatation of the left ventricle, which can lead to secondary dilatation of the mitral valve annulus. Dilation of the annulus spreads the mitral valve leaflets apart and creates poor tip coaptation and secondary leakage, or so-called "functional regurgitation."

Currently, primary regurgitation is corrected by attempting to remodel the native leaflets, such as with clips, sutures, hooks, etc., to allow them to close completely when the heart contracts. When the disease is too far advanced, the entire valve needs to be replaced with a prosthesis, either mechanical or biologic. Examples include suture annuloplasty rings all the way to actual valve replacement with leaflets, wherein the suture rings are sutured to the mitral valve annulus. Annuloplasty rings, which are also sutured to the annulus, have also been used to attempt to remodel the annulus, bringing the native leaflets closer together to allow them to properly close.

Based on the success of catheter-based aortic valve replacement there is growing interest in evaluating similar technologies to replace the mitral valve non-invasively using similar types of replacement valves.

Unlike the aortic valve, however, the mitral valve annulus does not provide a good landmark for positioning a replacement mitral valve. In patients needing a replacement aortic valve, the height and width of the aortic annulus are generally increased in the presence of degenerative disease associated with calcium formation. These changes in tissue make it easier to properly secure a replacement aortic valve in place due to the reduced cross-sectional area of the aortic annulus. The degenerative changes typically found in aortic valves are not, however, present in mitral valves experiencing regurgitation, and a mitral valve annulus is therefore generally thinner than the annulus of a diseased aortic valve. The thinner mitral valve annulus makes it relatively more difficult to properly seat a replacement mitral valve in the native mitral valve annulus. The general anatomy of the mitral valve annulus also makes it more difficult to properly anchor a replacement mitral valve in place. The mitral valve annulus provides for a smoother transition from the left atrium to the left ventricle than the transition that the aortic valve annulus provides from the aorta to the left ventricle. The aortic annulus is anatomically more pronounced, providing a larger "bump" to which a replacement aortic valve can more easily be secured in place.

In general, the aortic valve annulus is smaller than the mitral valve annulus. It has been estimated that the mitral valve annulus is about 2.4 cm to about 5 cm in diameter, while the aortic valve annulus has been estimated to be about 1.6 cm to about 2.5 cm in diameter.

The larger mitral valve annulus makes it difficult to securely implant current percutaneously delivered valves in the native mitral position. Current replacement aortic valves are limited in the amount of radial expansion they can undergo during deployment and implantation. To provide a replacement aortic valve that has an expanded configuration such that it can be securely anchored in a mitral valve annulus would require that the collapsed delivery profile of the replacement aortic valve be increased. Increasing the collapsed delivery profile, however, would make endovascular delivery more dangerous for the patient and more difficult to navigate the vasculature with a larger diameter delivery system.

Some attempts have been made to deliver and implant a one-piece replacement mitral valve, but it is difficult to provide a device that can be collapsed down to have a sufficiently small delivery profile and still be able to be expanded and secured in place within the mitral valve via a vascular access site.

A two-ring valve-support device suitable for endoscopic delivery was disclosed in a co-owned, co-pending US application (serial no. 13/224,124, filed on September 1, 2011 and published as US 2012/0059458). While this device provided technical solutions to most of the problems associated with prior art approaches (as described hereinabove), it was felt that in certain clinical situations it would be advantageous to be able to implant a valve-support which would be at least as stable as the above-mentioned two-ring support following implantation and which would also provide equal or superior support for a subsequently-implanted replacement cardiac valve, but which would be simpler in construction, have a smaller crossing profile and occupy less space within the heart cavity following implantation, US2011/319990 discloses an implant for supplementing, repairing, or replacing a native heart valve leaflet or leaflets. The implant provides a scaffold which defines a pseudo-annulus. The implant further has at least two struts in generally opposite spaced apart positions on the scaffold. The scaffold can be placed in an elastically loaded condition in a heart with the struts engaging tissue at or near the leaflet commissures of a heart valve annulus, to reshape the annulus for leaflet coaptation.

### SUMMARY OF THE INVENTION

The present invention is primarily directed to a prosthetic cardiac valve-support device comprising a single valve-support element (also referred to hereinbelow as the "support element") which is suitable for endovascular delivery to the region of an anatomical cardiac valve, wherein said support element is generally provided in the form of a single annular-shaped ring having an inner diameter and an outer diameter, said inner diameter defining the external boundary of an internal space, wherein said support element: has an outer perimeter that is entirely rigid; is fitted with one or more intra-ventricular and/or intra-atrial stabilizing elements; and has a collapsed delivery configuration and a deployed configuration, characterised in that said support element comprises one or more cut-out areas, which enable the inner perimeter of the single-ring support element to elastically deform in a radial direction.

Based on a consideration of the anatomical features of cardiac valves (particularly the mitral valve), an annular-shaped simplified, single-ring valve support device implanted on one side (preferably the superior side) of the annulus, or within the annulus itself would not intuitively be considered to be capable of being retained in position over a long period of time. However, it was unexpectedly found by the present inventors that said device was itself mechanically stable following implantation, and furthermore, was capable of providing a stable base for the subsequent implantation of a replacement valve (preferably a replacement aortic valve) within the central space of said annular support device.

In one embodiment, the support element is provided in the form of flat annular ring, preferably constructed from a material having superelastic and/or shape memory properties. One example of such a suitable material is Nitinol, which possesses both of the aforementioned physical properties. These properties may be utilized in order to permit said device, following its delivery in a collapsed conformation, to return to an expanded memory configuration after being heated above its transition temperature.

In the radial plane (i.e. the plane in which the native cardiac valve leaflets are disposed when in their closed position), the size of the annular support element may be defined in terms of its outer radius (*Ro*), its inner radius (*Ri*) and the difference between these two radii (*Rd*). It should be appreciated that *Ro* is determined by the diameter of the mitral valve annulus into which the valve support device will be implanted. *Ri*, however, is determined by the outer diameter of the replacement heart valve that will be inserted into the central space of the support device. Generally, the prosthetic aortic valves used in conjunction with the valve support device of the present invention have an external diameter considerably less than that of the mitral valve annulus. It may therefore be appreciated that *Rd* approximately corresponds to the annular gap between the small outside-diameter replacement valve and the relatively large diameter mitral valve annulus. Preferably, *Rd* is in the range of 1 - 14 mm. With regard to the thickness of the support element (*t*) (as measured along the longitudinal axis of the element when *in situ*), *t* represents a compromise between the need for minimizing this parameter in order to facilitate crimping and insertion into a delivery catheter, and the need for the support device to be sufficiently rigid such that it is able to withstand the forces exerted by the beating heart without buckling. In one typical, non-limiting example, *t* is 0.4 mm, while *Rd* has a value of 5.5 mm. Indeed, as a general rule, in most embodiments of the annular support element of the present invention, *R*d is significantly larger than *t*. For example, in many cases *Rd* may be between 2.5 and 35 times larger than *t*, more preferably between 10 and 20 times larger than *t*. It may be appreciated from the foregoing explanation that the ratio between *Rd* and *t* has functional significance for the valve support device of the present invention.

As indicated hereinabove, in a preferred embodiment of the invention, the valve support device is used to assist in the implantation of a prosthetic aortic valve into the mitral valve annulus of a human subject in need of such implantation. The thickness of the support device is generally in the range of 0.25 - 0.6 mm, more preferably 0.4 mm.

The annular support element may have an outline shape that is circular, elliptical or any other form that permits it to be adapted to make close contact with the inner cardiac wall upon implantation in the region of a cardiac valve annulus.

One particular feature of the valve support device of the present invention is the fact that the outer perimeter of the annular support structure is entirely rigid, such that in its deployed configuration, it is not possible to cause further expansion of the outer diameter of said device.

As mentioned hereinabove, unlike in the case of the aortic valve, the pathologically-involved mitral valve is generally not associated with increased calcification. One consequence of this lack of calcification is that it is not possible to increase stabilization of single-ring valve support devices within the mitral valve annulus by means of elements that exert their stabilizing forces in a radial direction. The reason for this is that he soft tissue of the uncalcified annulus in this situation would simply react to the applied radial forces by expanding in a radially-outward direction, thereby tending to reduce the contact between the support device and the tissue. Thus, in the case of the mitral valve, additional stabilization, if required, can only be achieved by means of stabilizing elements that apply forces on the heart tissues along the longitudinal axis. In this regard, it is to be noted that for the purposes of the present disclosure, the term 'radial' refers to the plane of the anatomical valve when the native leaflets are closed. The term 'longitudinal' refers to a direction that is at 90 degrees to the radial direction, namely approximately parallel to an imaginary line drawn from the cardiac apex to the cardiac base.

Thus, in some embodiments, the support element is fitted with heart tissue anchoring means adapted to securely anchor said support element to the heart wall. Non-limiting examples of such anchoring means include hooks and spirals.

The cardiac valve support further comprises one or more stabilizing elements, the function of which is to provide additional stabilization of said support within the ventricle and/or atrium. Preferably, the cardiac valve support is fitted with two or more stabilizing elements, more preferably two such elements attached to the support element such that the angular separation between them (measured around the circumference of the annular support element is approximately 180 degrees (+/20 degrees). This particular arrangement ensures that in use, the stabilizing elements can be positioned in the region of the medial and lateral mitral valve commissures. The advantage of such an arrangement is that the alignment of the support device stabilizing elements along the line of the native commissure ensures that said stabilizing elements will not interfere with the functioning of the native valve during the period prior to the deployment of a replacement valve within the central space of said support device.

The aforementioned stabilizing elements may be provided in any suitable form, including (but not limited to) additional complete ring structures, partial rings, curved arms or wings, elongate arms or wings and levered arms or wings. Examples of each of these types of stabilizing element are provided hereinbelow.

Thus, in some embodiments, the cardiac valve support comprises one or more intra-ventricular stabilizing elements, one or more intra-atrial stabilizing elements. In other embodiments, the cardiac valve support will be fitted with at least one intra-ventricular stabilizing element and at least one intra-atrial stabilizing element. Although in some cases, the stabilizing means include one or more elements that become physically attached to the cardiac tissue (e.g. in the atrial or ventricular walls), in many other embodiments, said stabilizing means provide additional mechanical stability by means of applying generally longitudinally-directed forces on the inner surface cardiac wall without being physically connected to the subsurface cardiac tissues.

In some other preferred embodiments, the stabilizing elements are provided in the form of elongate anchoring wings that are cut out of the same disc that is used to form the annular support ring itself. Said wings are used to anchor and stabilize the support device in its working location, by means of applying pressure to the inner ventricular wall. In most of the preferred embodiments described herein, the valve support devices comprise only two such anchoring wings. However, certain versions of the device may possess more than two wings.

In general, the anchoring wings of this embodiment are longer than the intra-ventricular and intra-atrial stabilizing structures disclosed hereinabove, and described in more detail hereinbelow. In many cases, this increased length of the anchoring wings is advantageous, since it allows said wings to make contact with a larger area of the ventricular wall surface, thereby resulting in improved stabilization of the support device.

In most preferred embodiments of this aspect of the invention, the single ring support structure contains only two wings, spaced apart by 180 degrees +/- a few degrees. The reason for this is that generally, the wings must be aligned along the mitral valve commissure in order to prevent hindrance of the native valve function during implantation of the replacement valve.

However, despite the need for the wings to be disposed opposite each other, most of the wing designs are asymmetric - that is, the two wings are not formed exactly opposite each other (i.e. exactly 180 degree separation), in order to avoid problems during crimping of the device prior to loading it into the delivery catheter. Rather they are arranged side-by-side when the disc is in a flat conformation (before the wings are bent downwards).

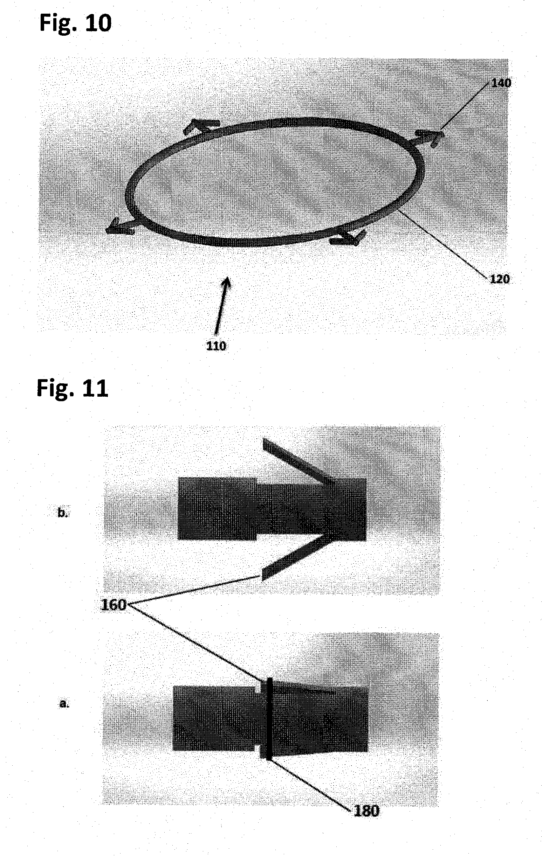

In a still further preferred embodiment, the stabilizing elements are provided in the form of lever-operated anchoring means, wherein said anchoring means comprise one or more anchoring arms and an equal number of fulcrums. Said anchoring arms and fulcrums are mutually disposed such that each anchoring arm is capable of being pivoted in a superior-lateral direction about its fulcrum upon application of a radially-outward force to said anchoring arm. Since this pivotable structure acts as a lever, it is thus able to apply significantly greater forces onto the ventricular wall than would be possible if the anchoring arm were constructed as an essentially static structure. In this way, this embodiment of the stabilizing elements of the present invention is capable of applying forces of sufficient magnitude to the ventricle, thereby enabling the single-ring support device to resist the strong displacement forces generated during ventricular systole. However, in order for lever structure of the present invention to function in its intended way, it is necessary to solve two further technical problems: firstly, the need to apply a sufficiently large force to the anchoring arms in order to cause them to pivot laterally and, in turn, exert similarly large forces on the inner ventricular wall, and secondly the need to time the generation of this laterally-directed expansive force such that said force is applied only when needed - that is, during the second stage of the two-stage replacement valve implantation procedure. In order to explain this second point, it is necessary to briefly consider the manner in which the mitral valve normally functions. Thus, during early systole, the intra-ventricular pressure increases to a point such that the forces that are thereby exerted on the mitral valve leaflets are sufficient to cause them to close, thereby preventing retrograde flow of blood from the left ventricle into the left atrium. During the first stage of the two-stage procedure (implantation of the support structure), the support device is not subjected to strong displacing forces during ventricular systole, due to the closure of the native leaflets, resulting in complete (or near-complete) separation between the left ventricle and left atrium. Furthermore, even when the native leaflets are open (during diastole) the valve support device is subjected to only very low pressure since firstly, the surface area of the ring-shaped support device is small, and secondly, most of said surface area is not situated in the path of the fluid flow between the atrium and ventricle. However, during the second stage of the two-stage method (implantation and expansion of the replacement valve), the native mitral valve leaflets become displaced laterally. Said leaflets are, in this way, prevented from closing during early systole. Consequently, during ventricular systole, very strong upwardly-directed forces are exerted on the replacement valve leaflets, thereby causing them to close. Since the replacement valve leaflets now form a single structure, together with the valve support device, the forces acting on said replacement valve leaflets would cause displacement of the attached valve support device were it not strongly anchored to the ventricular wall. Thus, it is during this second stage of the implantation procedure that it is essential that the anchoring arms of the support device are able to strongly engage with the ventricular wall, in order to counter the sudden increases in the displacing forces applied to said device.
The two aforementioned technical problems have been solved in an ingenious manner by the present inventors by means of exploiting the radial expansion of the replacement valve (during the second stage of the two-stage implementation method), either by means of an inflatable balloon or by the use of a self-expandable stent. In this way, the outwardly-radial forces exerted by the balloon (or self-expanding stent) are transferred to the medial portion of each of the anchoring arms in the support device. Said arms are then caused to pivot about their fulcrum (more details concerning which will be provided hereinbelow). This pivoting motion then continues until the lateral portion of each anchoring arm makes contact with the ventricular wall (or, in some embodiments, together with the medial portion, causes pinching of the native valve leaflets, or in other embodiments come in contact with the device lateral attachment wings (shown in Fig. 10 - thus increasing the axial force attaching the wings to the left ventricle and increasing the anchoring force of the wings). In summary: the forces applied by the expanding replacement valve cause radial expansion of the anchoring arms. As a result of the lever arrangement of said arms, the magnitude of the radially-directed force generated by the expanding valve is amplified. An additional benefit derived from the pivotable arm arrangement is that the angle formed between the lateral extremity of each expanded anchoring arm and the tissue of the ventricular wall becomes altered, such that said arm transfers said radially-directed force to said tissue in an axial direction (that is, along the longitudinal axis of the free lateral extremity of the anchoring arm). This directional effect is highly advantageous, since the geometry of anchoring arms is such that they are able to apply greater forces on the heart wall in the direction of their longitudinal axis than if said forces were to be applied at 90 degrees to said axis. In this regard, it should be noted that the aforesaid directional effect does not require that it is the free end of the anchoring arms that make contact with the tissue. Indeed, in certain circumstances such an arrangement may prove undesirable since it may result in trauma to the ventricular tissue. Rather, it is sufficient that a short length of the terminal (i.e. lateral-most) portion of the anchoring arm is angled, thereby forming a non-traumatic base. In such an arrangement, most of the forces exerted by the ventricular wall onto the anchoring arms are still directed axially (i.e. in a longitudinal direction), and thus buckling of the said arms is prevented. Finally, the fact that the force-generating step is the expansion of the replacement valve results in the high-magnitude forces being applied by the anchoring arms at exactly the right moment - that is, from the moment when the native valve leaflets have become immobilized. Preferably the anchoring arm is constructed such that it is bent at a point along its length, such that said arm may be considered to comprise a medial portion and a lateral portion, wherein said portions form an angle greater than 0 degrees between them. It is to be noted that this angle may become larger or smaller during the pivoting movement of the anchoring arms. However, in one preferred embodiment, the angle is progressively reduced to almost 0 degrees (i.e. nearly complete closure of the lateral portion on to the medial portion, as the lateral expansion continues towards its endpoint. Further details of this pivotable-arm embodiment will be described hereinbelow.

In some embodiments the support element is fitted with replacement valve engagement means adapted to securely engage a replacement heart valve. In some embodiment, the engagements means can have anchoring and/or locking elements adapted to securely lock with a portion of a replacement heart valve. In other embodiments, the replacement valve engagement means are formed from a soft biocompatible material (such as a biocompatible fabric, silicon, PET etc.) which is fitted to the external surface of portions of the support element. In these embodiments, the soft, compressible nature of the biocompatible material permits certain portions thereof to be compressed by the struts or other structural elements of the replacement valve, upon expansion within the lumen of the valve support. Other portions of the soft biocompatible material which are not compressed by the expanded replacement valve protrude into the internal space of said valve between the struts and/or other structural elements. The protrusions formed in this way engage and grip the replacement valve thereby preventing its movement in relation to the valve support. In other embodiments, the replacement valve engagement means comprise rigid anchors of a size and shape such that they are capable of entering the internal space of the replacement valve between its struts and/or other structural elements, upon expansion of said valve within the internal space of the valve support.

According to the present invention, the inner perimeter of the annular single-ring support element is able to elastically deform in a radial direction. In this regard, it is to be appreciated that the annular shaped elastically-deformable support element of the present invention possesses an external diameter and an internal diameter. In use, a support element of the present invention is selected such that the replacement cardiac valve, in its fully expanded, deployed configuration, has an external diameter which is slightly larger than the inner diameter of said support element when the latter is at rest. Then, upon deployment of the replacement valve within the internal space of the valve support element, the internal diameter of said support element, by virtue of its elastically-deformable nature, is increased. The replacement valve is thus held firmly in place within the support element by means of the radially-inward forces that are exerted by said support element on said valve as a result of the tendency of the elastic inner surface of said support element to return to its rest position.

It is to be noted that the elastically-deformable support element may be constructed such that either its entire inner surface is elastically deformable or, alternatively, it may be constructed such that only certain discrete regions thereof are elastically deformable.

Currently available Aortic valves which may be implanted via a trans-femoral approach or a trans-apical approach are generally made either of balloon expandable material (for example Stainless steel - such as Sapien stented valve, Edwards Inc.) or self-expanding material (for example Nitinol - such as the CoreValve stented valve, Medtronic inc., and Lotus stented valve, Sadra, Boston Scientific Inc.).

In balloon expandable valves, after deployment and expansion of the valve in its position the stent of the valve has a recoil phenomenon. This means that immediately after maximal balloon expansion, when the balloon is deflated, there is some recoil, some "closing back" of the stent. This effect is a physical mechanical property of the metallic balloon expandable stent. When implanted in the Aorta, the Aortic wall is elastic, and after its expansion it applies an inward force on the stent, maintaining it in position. However, if one inflates such a stented valve in a completely rigid tube/element than immediately after expansion the stent will have some recoil, but the rigid element will not have any recoil due to its rigidity - hence there will always be some space between the stent and the rigid element, and the stent will not be held in its place by strong forces.

Self-expanding stents do not display recoil phenomena; however they present a different challenge for deployment within a valve-support. The externally directed forces applied by self-expanding stents is lower than that of balloon expandable stents, hence it is a significant challenge to ensure that a self-expanding stented valve will be deployed and secured into a valve-support, without being dislocated during the cardiac cycle. Hence there is a significant advantage if the valve support has one or more support elements which are capable of applying internally directed radial forces, which increase the forces attaching the valve to the valve support and ensure the valve will not be dislocated.

In some further preferred embodiments of the invention, at least one segment of the single-ring annular support element has an external diameter that is larger than the diameter of the cardiac annulus into which said support element will be implanted (hereinafter referred to as "enlarged diameter segments" or similar), and wherein at least one other portion of said annular element has an external diameter that is smaller than the diameter of said annulus (hereinafter referred to as "reduced diameter segments" or similar). It may thus be appreciated that said reduced diameter segments interrupt the otherwise-ring shaped outer circumference of the support element of the present invention.

In one preferred embodiment of the present invention, the support element has two enlarged diameter segments and two reduced diameter segments. In another preferred embodiment the support element comprises four enlarged diameter segments and four reduced diameter segments.

It will be appreciated that, upon implantation of the cardiac valve support element of the present invention into the region of the cardiac annulus, each of the enlarged diameter segments will form a fluid-tight seal against the tissue of the anatomic annulus. Conversely, a small aperture will be created between each of the reduced diameter segments and the adjacent portion of the annulus, thereby permitting the limited peri-valvular flow of blood between the ventricle and atrium on the side of the heart in which said support element is implanted. In this way, the fluid pressure (and hence force) exerted by the contracting heart on the cardiac valve support (and on a replacement valve situated within said support) will be reduced. Additionally, this unique design reduces the afterload against which the ventricle contracts, since it allows a controlled limited regurgitation, and thus may have beneficial clinical effects on ventricular function, reducing ventricular wall stress and oxygen consumption.

In some embodiments the support element has at least one coupling element adapted to reversibly couple to a delivery system. The at least one coupling element can be a threaded bore.

In a further preferred embodiment of the present invention, the annular support element is fitted with at least one wing-like drape element made of a biocompatible fabric. The drape functions as a sealing element, positioned between the support element and the mitral annulus, thereby preventing paravalvular leakage after implantation of the valve support in the mitral annulus.

In one preferred embodiment of this aspect of the invention, a single drape element is attached to the entire circumference of the annular support element. In another preferred embodiment, a plurality of drapes is attached to the support element. In this embodiment, the drapes may be positioned such that there is a small overlap between adjacent drapes, thereby preventing leakage therebetween. This drape sealing feature uniquely solves the problem of paravalvular leakage following implantation of the replacement valve, since the fabric drape element is capable of moving in response to force applied by the blood flowing during ventricular systole such that it seals most or all of the residual space between the support element and the mitral (or other) valve annulus.

One aspect of the disclosure is a system adapted for endovascular or transapical delivery to replace a mitral valve, comprising: a prosthetic cardiac valve support device comprising a single ring-shaped support element with a collapsed delivery configuration and a deployed configuration, as disclosed hereinabove; and a replacement heart valve comprising an expandable anchor and a plurality of leaflets, wherein said replacement heart valve is adapted to be secured to the prosthetic cardiac valve support device.

In a highly preferred embodiment, the replacement heart valve is a prosthetic aortic valve.

In some embodiments the support element is adapted to securingly engage the replacement heart valve. In one such embodiment, the replacement valve securing means comprise attachment means, such as hooks or other mechanical anchors that are connected, at one of their ends, to the support element, and have a free end for attachment to the replacement valve.

In some embodiments of the invention, the system disclosed hereinabove further comprises pressure measuring elements. These elements may be situated anywhere in the system - including on the surface of the valve support device, attached to the replacement valve, as well as within the guide catheter. In another embodiment, the system of the invention further comprises connection terminals that permit the connection of pacemaker leads to various parts of said system.

One aspect of the disclosure is a method of replacing a patient's mitral valve, comprising: delivering a valve support device to a location near a subject's mitral valve, the valve support device being one according to the invention; and expanding the support element from a collapsed configuration to a deployed configuration secured against cardiac tissue above the plane of the mitral valve annulus, below this plane or against the annulus itself;

In one aspect of the disclosure, the above-defined method may be employed to deliver the valve support device by an endovascular route. In another aspect of disclosure, the method may be used to deliver the valve support device by a transapical route.

In some aspects of disclosure expanding the support element comprises allowing the support element to self-expand against cardiac tissue.

In certain aspects of disclosure, the method further comprises the step of causing cardiac attachment means fitted to the support element to become inserted into the ventricular wall. In certain cases, the insertion of said attachment means is effected by means of control wires inserted through the delivery catheter which are used to cause rotation of the valve support device. In other cases, said attachment means may be covered by a sleeve during insertion of the valve support device, said sleeve being removed in order to allow said attachment means to become inserted into the ventricular wall. In still further aspects of disclosure, the attachment means may be constructed in the form of an anchor with two or more backwardly-pointing self-opening distal arms, wherein said distal arms are retained in a closed conformation by means of a resorbable suture. Then, after a certain period of time following insertion of said attachment means into the ventricular tissue (e.g. between a few hours and few weeks), said suture dissolves, thereby permitting the distal arms to adopt their open conformation.

In other aspects of disclosure, the above-defined method further comprises the step of causing intra-ventricular stabilizing elements and/or intra-atrial stabilizing elements to engage, respectively, the inner ventricular wall and/or inner atrial wall.

In some aspects of disclosure, expanding the support element comprises expanding the support element towards a generally annularly shaped deployed configuration.

In some embodiments expanding the support element comprises expanding the support element secured against papillary muscles and chords attached to the native mitral valve, and can be done without displacing them.

In some aspects of disclosure, the method further comprises securing a replacement heart valve to the valve support. Securing the replacement heart valve to the valve support can comprise expanding the replacement heart valve from a collapsed delivery configuration to an expanded configuration. Expanding the replacement heart valve can include expanding the replacement heart valve with a balloon and/or allowing the replacement heart valve to self-expand. Securing a replacement heart valve to the valve support can comprise securing the replacement heart valve radially within the valve support. Securing a replacement heart valve to the valve support can comprise locking a replacement heart valve element with a valve support element to lock the replacement heart valve to the valve support. In other aspects of disclosure, the step of securing a replacement valve to the valve support device comprises causing valve attachment means fitted to the valve support element to engage said replacement mitral valve. The replacement valve may be delivered by either an endovascular route or by the transapical route.

In one aspect of disclosure of this method, the valve support device and the replacement heart valve are delivered by the same route.

In a further aspect of disclosure, the above-disclosed method to deliver a valve support device and a prosthetic heart valve may combine two separate delivery approaches - one approach for the support device and a different one for the valve. The advantage of this strategy is that it significantly shortens the time delay between deployment of the valve anchor and the deployment of the prosthetic valve itself. This is important, since after deployment of the valve support there may be interference with the native mitral valve function (due to interference with the valve leaflets). One example of such an approach is the delivery of a valve support *via* an endovascular, trans-septal route (as described herein), while in parallel delivering the prosthetic mitral valve *via* a transapical or transfemoral route (as known in the art). Conversely, the valve support may be delivered by a transfemoral or transapical approach, while the replacement valve itself is delivered trans-septally. Thus, in one aspect of disclosure of the method disclosed above, the replacement mitral valve is delivered by the same route as the valve support. In another embodiment of the method, the replacement mitral valve and the valve support are delivered by different routes, wherein said routes are selected from the group consisting of trans-septal, transfemoral and transapical. The use of these various approaches to delivery replacement valves and other devices is well known to the skilled artisan and has been described in several publications including US 7,753,923 and WO 2008/070797.

In a preferred aspect of disclosure of the method disclosed above, the replacement heart valve used to replace the native mitral valve is a prosthetic aortic valve. Examples of suitable prosthetic aortic valves include (but are not limited to) the following commercially-available replacement valves: Sapien Valve (Edwards Lifesciences Inc., US), Lotus Valve (Boston Scientific Inc., US), CoreValve (Medtronic Inc.) and DFM valve (Direct Flow Medical Inc., US).

As mentioned hereinabove, the method of the disclosure is a two-step method for replacing a native cardiac valve, preferably the mitral valve, with a prosthetic valve, wherein the first stage comprises deploying a single-ring valve support device according to the invention in the region of the native mitral annulus, and the second stage comprises the expansion of an expandable prosthetic valve within the central space of said support device. One of the key advantages of the use of the valve support device of the present invention in this method is that its shape, size and the disposition of its stabilizing arms all permit the native cardiac valve leaflets to continue functioning in the time interval between the deployment of said valve support device (i.e. the first step of the procedure) and the deployment of the replacement valve (i.e. the second step of the procedure). A further key advantage of the presently-disclosed valve support device is that its flat annular form allows the dysfunctional mitral valve to be replaced by a commercially-available prosthetic aortic valve. This is achieved by virtue of the fact that the annular support device is able bridge the gap between the relatively small diameter prosthetic aortic valve and the relatively large diameter native mitral valve annulus. Further advantages of the present invention will become apparent as the description proceeds.

### BRIEF DESCRIPTION OF THE DRAWINGS

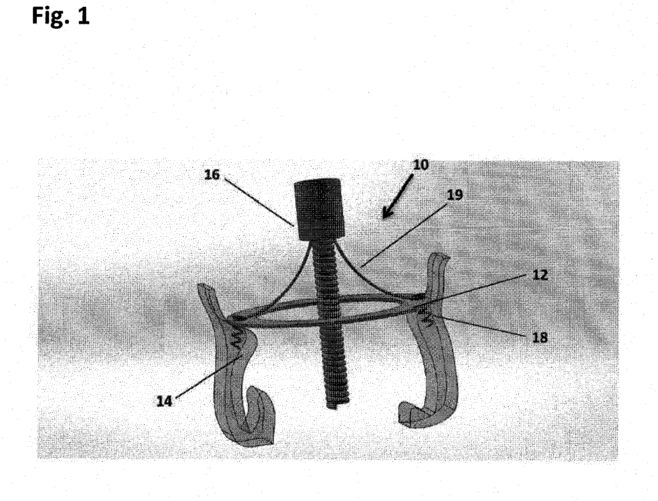

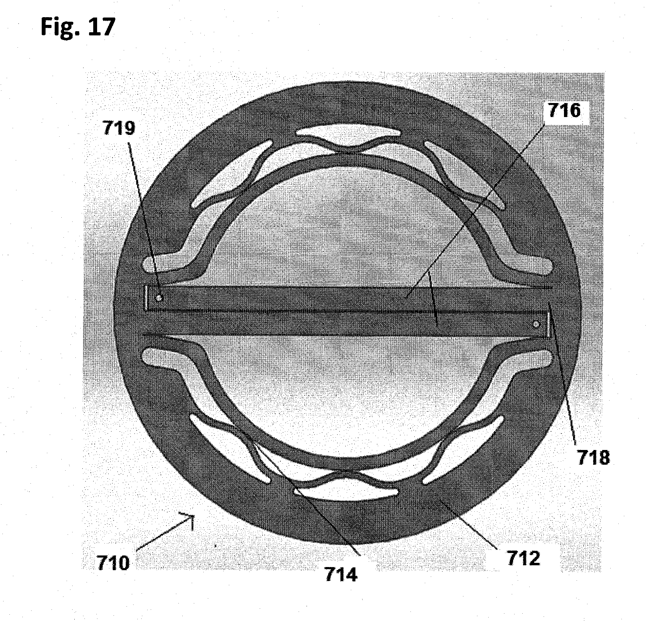

The novel features of the disclosure are set forth particularity in the appended claims. A better understanding of the features and advantages of the present disclosure will be obtained by reference to the following detailed description that sets forth illustrative embodiments, in which the principles of the disclosure are utilized, and the accompanying drawings, of which Figures 1 to 16 are for illustration only and Figures 17 to 49 are examples of the present invention:
Figure 1 depicts a single-ring valve support containing spiral-shaped cardiac anchoring means.
Figure 2 depicts two support elements, each having the same internal diameter but different external diameters.
Figures 3A and 3B illustrate wherein the valve support is fitted with one horizontal stabilizing element (A) and one vertical stabilizing element (B).
Figures 4A and 4B depict wherein the valve support each having a stabilizing element formed from a stent-like mesh.
Figure 5 illustrates wherein the valve support in which the stabilizing element contains spring-like constricted regions.
Figure 6 depicts wherein the valve support having a plurality of stabilizing elements attached to the support element.
Figures 7A - 7C depict wherein the valve support of the present invention in which the stabilizing elements are constructed in the form of curved arms.
Figure 8 illustrates wherein the valve support in which the support element is connected to a horizontal ring-shaped stabilizing element.
Figures 9A and 9B show a valve support device with a pair of elastic tab-like stabilizing elements attached to the support element.
Figure 10 depicts a valve support comprising a plurality of hook-like cardiac anchors.
Figures 11A - 11B illustrate cardiac attachment anchors having backwardly pointing distal arms which may be retained in a closed position during delivery by means of a resorbable suture loop.
Figures 12A - 12B illustrate two different cover elements that may be used to conceal the cardiac attachment anchors during delivery of the valve support.
Figures 13A - 13B depict the use of a shape-memory anchor which is maintained in a straight conformation during delivery by means of an overtube.
Figure 14 illustrates clip-like cardiac tissue anchors that are particularly suitable for attaching the support element to the annulus.
Figures 15A - 15B illustrate support elements fitted with a valve engagement means constructed from a soft biocompatible material.
Figure 16 illustrates an exemplary delivery system for delivering a replacement mitral valve support structure.
Figure 17 illustrates a support device fitted with two elongate anchoring wings.
Figure 18 shows the support device of Fig. 17 after the wings have expanded into their open, working position.
Figure 19 illustrates the valve support device of Figs. 17 and 18 following its implantation into the heart in the region of the cardiac annulus.
Figure 20 depicts a different embodiment of the invention, wherein the anchoring arms have enlarged basal sections.
Figure 21 shows the embodiment of Fig. 22 in its expanded conformation.
Figure 22 depicts a different embodiment, wherein the anchoring wings are broader than in the previously depicted embodiments.
Figure 23 depicts a device having two short wings and two long wings.
Figure 24 shows a support device fitted with an open-work structure.
Figure 25 depicts an embodiment having an alternative open wing structure.
Figure 26 illustrates an example of a first implementation of the levered-operated wing embodiment in its expanded conformation.
Figure 27 shows a similar embodiment to that shown in Fig. 26, but in its pre-expanded conformation.
Figure 28 provides an enlarged view of a fulcrum point in one embodiment of the lever-operated wings.
Figure 29 shows a device of the invention fitted with a second implementation of the lever-operated wings in its fully expanded conformation.
Figure 30 shows the embodiment of Fig. 29 in its pre-expanded conformation.
Figure 31 provides a perspective view of another embodiment of the second implementation of the lever-operated wings.
Figure 32 illustrates a third implementation of the lever-operated wings of the present invention.
Figure 33 depicts a "leaflet pinching" embodiment of the third implementation of the lever-operated wings.
Figure 34 shows an embodiment comprising both static and levered arms, prior to expansion.
Figure 35 shows the embodiment of Fig. 34 following expansion of the replacement valve.
Figure 36 illustrates a top view of an exemplary support element showing the elastic inner perimeter feature.
Figure 37 illustrates another top view of another exemplary support element fitted with elastically deformable elements.
Figure 38 illustrates a perspective view of another exemplary support element of the invention fitted with elastically deformable elements.
Fig. 39 illustrates a perspective view of an exemplary replacement valve support fitted with pressure release means, in an expanded configuration.
Figure 40 provides an illustratory side view of an exemplary support of the present invention, shown in a position on the mitral annulus, and exemplifying a fabric drape attached to the inner part of the ring.
Figure 41 illustrates a side view of another exemplary upper ring (upper support element) of a valve support of the invention, shown in a position on the mitral annulus, and exemplifying a fabric drape attached to the outer (distal) part of the ring.
Figure 42 illustrates a perspective view of an exemplary upper ring (upper support element) of a valve support of the invention, exemplifying multiple fabric drapes of the invention.
Figure 43 illustrates an exemplary design of a fabric of a drape of the invention.
Figure 44 shows a single-ring support device intended for use in conjunction with a self-expanding aortic valve.
Figure 45 shows a single-ring support device intended for use in conjunction with a balloon-expandable aortic valve.
Figures 46 - 49 are photographs showing the successful implantation of a single-ring support device of the present invention into a cadaveric heart.

### DETAILED DESCRIPTION OF THE INVENTION

The disclosure is generally related to cardiac valve support structures that are adapted to be implanted near or within a native cardiac valve or native valve annulus and are adapted to provide support for a replacement heart valve. The support structures are adapted to interact with a replacement heart valve to secure it in an implanted position near or within the native valve or native valve annulus. In some embodiments the support structure is adapted to be positioned near or within the mitral valve annulus, and is adapted to interact with a subsequently delivered replacement mitral valve to secure the replacement mitral valve in place to replace the function of the native mitral valve.

The disclosure also provides for two-step endovascular implantation procedures for replacing a patient's native mitral valve. In general, a support structure is first positioned near or within a mitral valve annulus and secured in place. A replacement mitral valve is subsequently secured to the support structure, securing the replacement valve in place near or within the annulus. By implanting the support structure and replacement mitral valve in two steps, the replacement mitral valve can have a lower delivery profile because it does not have to expand as much to contact native tissue due to the presence of the support structure. This eliminates the need to have a large delivery profile replacement valve as would be required if attempting to position an aortic valve in the native mitral valve, or if attempting to position a one-piece mitral valve implant (i.e., an implant not assembled in-vivo) within the native mitral valve.

Fig. 1 illustrates a valve support device in an expanded configuration, following its delivery through a guide catheter and implantation at its target site. Thus, Fig. 1 shows a guide catheter **16** that was used to deliver a valve support device **10** of the present invention, wherein said device comprises a single ring-shaped support element **12.** At the stage of the delivery process shown in this figure (which will be described in more detail hereinbelow), said support elements **12** has self-expanded into its working conformation.

In some embodiments the support element is generally annular in shape in its expanded configurations (illustrated in Fig. 1). Patient-to-patient variability in the cardiac anatomy can, however, require that the support elements have a variety of sizes and configurations. The support elements can therefore have any configuration as needed to be secured to any anatomical configuration. For example, the support elements can have generally elliptical configurations.

In some embodiments the support element is made from a resilient material that can be deformed into a delivery configuration yet is adapted to self-expand to an expanded configuration, with optional additional expansion by balloon dilation. For example, the support can be made from Nitinol, relying on its superelastic properties. In some embodiments the valve support is made from a material with shape memory properties, such as nitinol, and is adapted to return to an expanded memory configuration after being heated above its transition temperature. In some embodiments in which the valve support is made from a material such as nitinol, the shape memory properties and the superelastic properties are utilized.

Once the support structure is expanded and secured in place within the native mitral valve, a replacement mitral valve in a collapsed delivery configuration is advanced and positioned within and below the support structure. The replacement mitral valve is then expanded (e.g., by balloon expansion, self-expansion, etc.), thereby causing the replacement mitral valve to engage with, and become secured by, the single-ring support element.

Further details of exemplary deployment procedures are described below.

As disclosed hereinabove, in some embodiments of the support element may be constructed from an annular ring; such a ring may be manufactured from sheets of the shape-memory / superelastic material. In other embodiments, however, the support element is constructed from a shape memory/superelastic wire (such as a Nitinol, cobalt or stainless steel wire). One advantage of this design is the fact that the use of wire results in low manufacturing costs. A further significant advantage is that the use of a single wire (rather than a broader strip - as depicted in Fig. 1) is that it may be collapsed to a very small size such that it may be inserted into a small diameter delivery catheter, thereby presenting a reduced crossing profile.

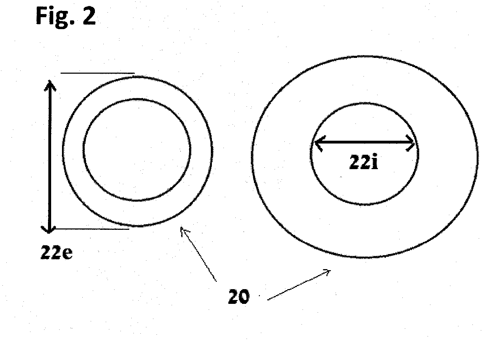

In the embodiment of the valve support disclosed herein in which said support is constructed in the form of an annular ring (such as shown in Fig. 1), the size of the ring-like support element may, as depicted in Fig. 2, be defined by two different dimensions - an external diameter **22e** and an internal diameter **22i.** It will be seen that while both of the support elements **20** shown in this figure have the same internal diameter, their external diameters differ. It will be appreciated that the internal diameter defines the space available for implantation of the replacement valve within the valve support device, while the external diameter needs to be the same as the space within the native Mitral annulus (in order to permit stable implantation of the valve support). Since both the expanded diameter of different commercially-available replacement mitral valves and the diameter of the anatomical mitral annulus differs (from patient to patient), it follows that a range of valve support devices needs to be manufactured and made available, such that the clinician can select the valve support having an internal diameter appropriate for the replacement valve to be implanted and an external diameter of the same size as the space within the mitral annulus.

In some illustrations, the support elements do not have a covering element. In other illustrations, however, the support element can have a covering element such as a sealing skirt to enhance the sealing of blood flow in and around the support structure and replacement heart valve. The covering element can be any type of material that surrounds the support element and provides the enhanced sealing functionality (e.g. it can prevent fluid leakage between the valve support and the heart wall). In some cases, the covering element can be attached (e.g. by the use of a biocompatible adhesive) to the outer surface of the support element. In other cases, the covering element can be attached to the inner surface of the support element.

In some embodiments one or more of support structures is covered in a material such as a polyester fabric (e.g., Dacron).

The valve support device may further comprises one or more stabilizing elements attached to the support element. The purpose of the stabilizing elements is to increase the multidirectional stability of the implanted valve support device (and thus also enhance the stability of the implanted replacement valve), by means of stabilizing elements in the form of additional complete ring structures, partial rings or curved arms, elongate arms or wings and levered arms or wings, whereby said structures are placed such that at least part of their length is in close apposition to the surface of the inner ventricular wall and/or the surface of the inner atrial wall. Since the curvature of the inner walls of both the atrium and ventricle may be defined in relation to two mutually-perpendicular axes (horizontal and vertical), the stabilizing elements may be disposed either horizontally (i.e., essentially parallel to the horizontal axis of the valve support device) or vertically (i.e. essentially parallel to the vertical axis of the valve support device.). Additionally, in some embodiments, the stabilizing elements may be disposed such that they are neither parallel to the horizontal axis nor to the vertical axis, but rather are arranged at an acute angle to one of these axes.

In some cases, the stabilizing elements (which may be formed from either elastic or plastic materials, as will be described hereinbelow) will be manufactured as an integral part of the valve support device. In other cases, said stabilizing elements will be manufactured separately (by casting, milling, laser-cutting or any other suitable technique known to skilled artisans in the field), and later connected to one or both support elements by means of soldering or laser welding.

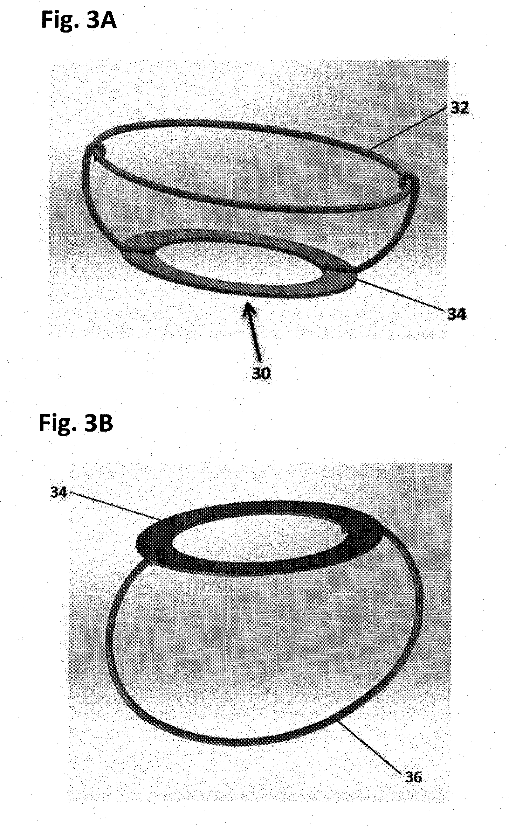

Fig. 3A illustrates a valve support device **30** fitted with a single, horizontally-disposed ring-shaped stabilizing element. As shown in the figure, the upper, apical stabilizing element **32** is attached at its lower portion to the support element **34.** Fig. 3B depicts another valve support device in which a single, vertically aligned ventricular stabilizing element **36** is attached at its lower portion to support element **34.** In use, the upper stabilizing element shown in Fig. 3A will be in contact with the inner atrial wall, while the lower stabilizing element of Fig. 3B will contact the inner ventricular wall.

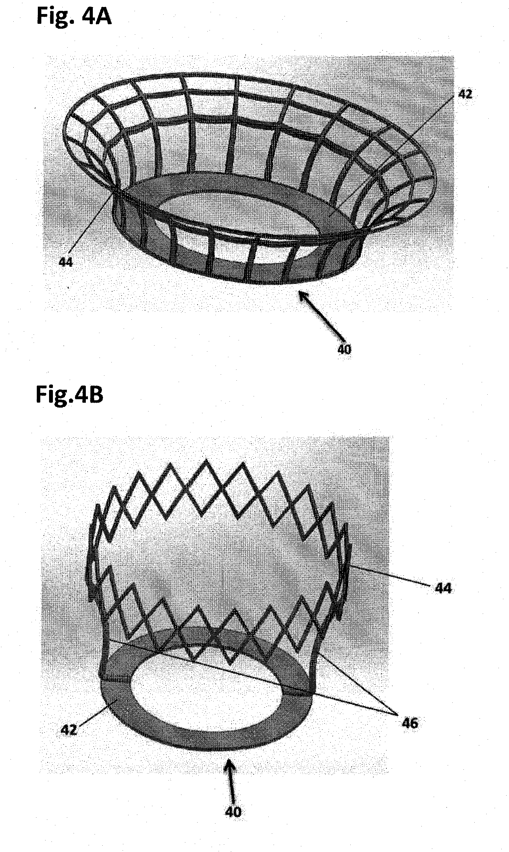

In the case of horizontal stabilizing elements, the element itself can (as explained above) be a complete ring, a partial ring or a curved elongate arm. While in some complete ring embodiments, the stabilizing element is constructed from a single looped wire or solid band, in other embodiments, it may be constructed in the form of a stent-like mesh. Fig. 4A illustrates one embodiment of this type, in which the mesh-like stabilizing element **44** is attached directly to the support element **42** of valve support device **40.** Alternatively, as shown in Fig. 4B, the mesh-like stabilizing element **44** may be connected to the support element **42** by means of spacer arms **46,** which serve to increase the separation distance between the stent-like mesh stabilizer **44** and said support element **42.**

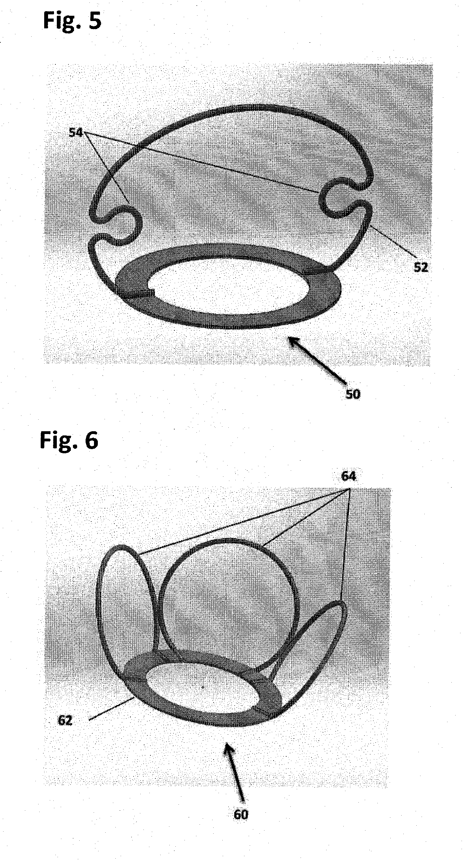

While the stabilizing element is generally constructed such that its outline shape is that of a smooth curve, in another option, as depicted in Fig. 5, this smooth curve is broken by one or more constricted regions **54,** wherein said regions act as spring-like elements, increasing the force that the stabilizing element **52** is capable of applying to the inner ventricular or atrial wall, and thereby enhancing the ability of said stabilizing element to stabilize the valve support device **50.** The device shown in Fig. 5 contains a single, upper (apical) vertical stabilizing element. However, in other versions of this option , the valve support device may be fitted with one or more vertical stabilizing elements and one horizontal stabilizing elements attached to the other support element. In some other embodiments, the valve support device contains only one such stabilizing element (horizontal, vertical or otherwise angled). In still further embodiments, a single valve support device may contain one stabilizing element containing one or more constricted regions **54,** as shown in Fig. 5, together with one or more stabilizing elements of any of the other types disclosed and described herein.

All possible combinations of the various types of stabilizing element disclosed herein may be used, as appropriate. It should also be noted that more than one stabilizing element may be attached to the support element. Fig. 6 illustrates one embodiment of this type, in which the support element **62** of the valve support device **60** is fitted with several (in this case, three) non-horizontal, angled, atrial stabilizing elements **64.**

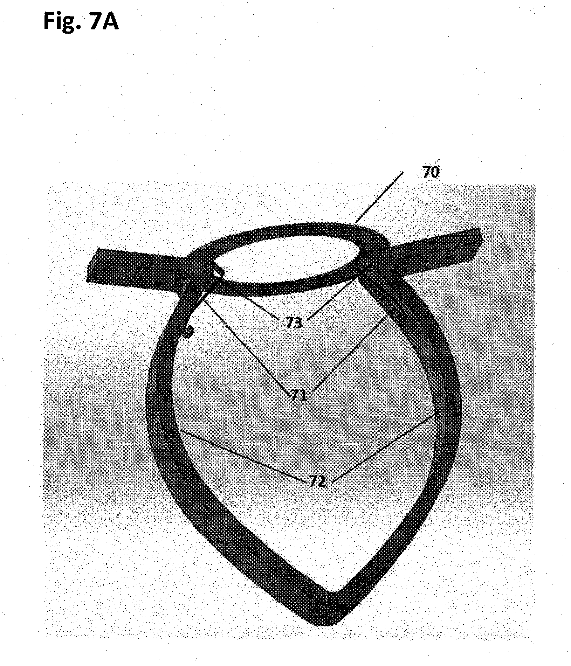

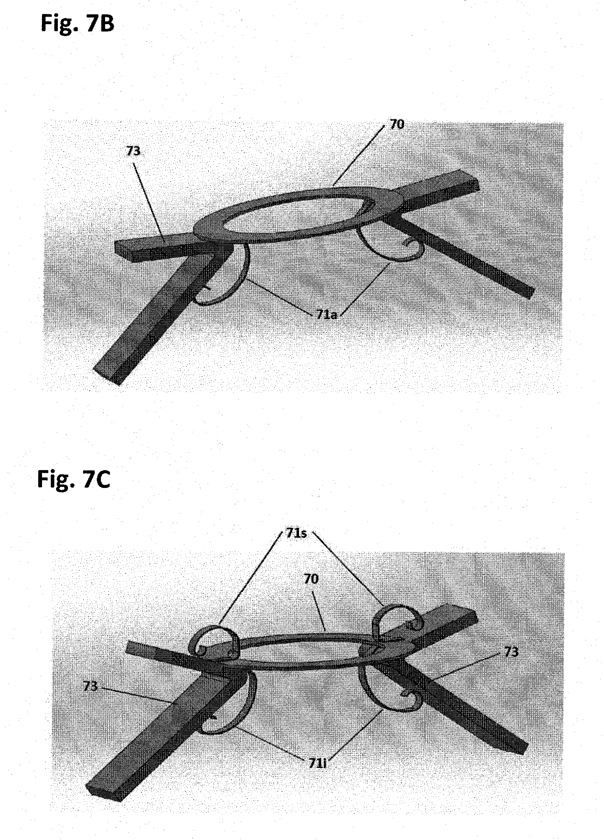

As explained hereinabove, the stabilizing element need not be provided in the form of a complete ring, but rather may also have the form of a partial ring or a curved elongate arm. Various examples of the latter type of stabilizing element are shown in Figs. 7A, B and C. Thus, Fig. 7A depicts a support element **70** of a valve support device, wherein said valve support device is connected to - and stabilized by - two curved elongate arms **71** which are disposed vertically downwards along the inner ventricular wall **72.** In the example shown in this figure, the stabilizing elements **71** are constructed from an elastic material (such as cobalt base alloy, nitinol, stainless steel and other biocompatible metals and metal alloys). The curved arms typically have a length of between 1 mm and 50 mm, preferably about 20 mm. As will be seen in the figure, the upper part of each stabilizing element **71** is angled such that it is able to pass around the cardiac annulus **73.** In some embodiments, the elongate, curved elastic arms may be constructed such that they are in a state of pre-load. The elastic properties of the stabilizing elements will cause said element to tend to both grip the annulus and to apply an outward force on the ventricular wall inferior to the annulus. In an alternative of this aspect of the device of this invention, the curved elongate stabilizing elements may be constructed from a plastically-deformable material such as stainless steel, cobalt base alloy and nitinol. In this case, the elongate arms are molded around the annulus using a clenching or crimping tool. In this way, the upper sections of the elongate arms will firmly grip the annulus, while the lower sections will be biased outward and downwards along the ventricular wall.

Fig. 7B illustrates another alternative of this aspect of the device, wherein the stabilizing elements **71a** attached to support element **70** are much shorter than those shown in Fig. 7A, and apply a stabilizing force to the inferior surface of the annulus **73** (rather than to the lateral inner walls of the ventricle). During implantation, the stabilizing elements are brought into position below the annulus, such that the annulus becomes "trapped" between said stabilizing elements and the support element itself.

A still further variant of this aspect is illustrated in Fig. 7C. This variant differs from the aspect shown in Fig. 7B, in that the support element **70** is fitted with both upper (**71s**) and lower (**71i**) stabilizing elements. During implantation into a patient, the valve support device is manipulated such that the annulus **73** becomes "trapped" between these upper and lower stabilizing elements. In each of the variants of this embodiment, the short stabilizing elements may be brought into position by means of a balloon expansion mechanism, by a mechanical closure mechanism or, alternatively, said stabilizing elements may be self-expanding.

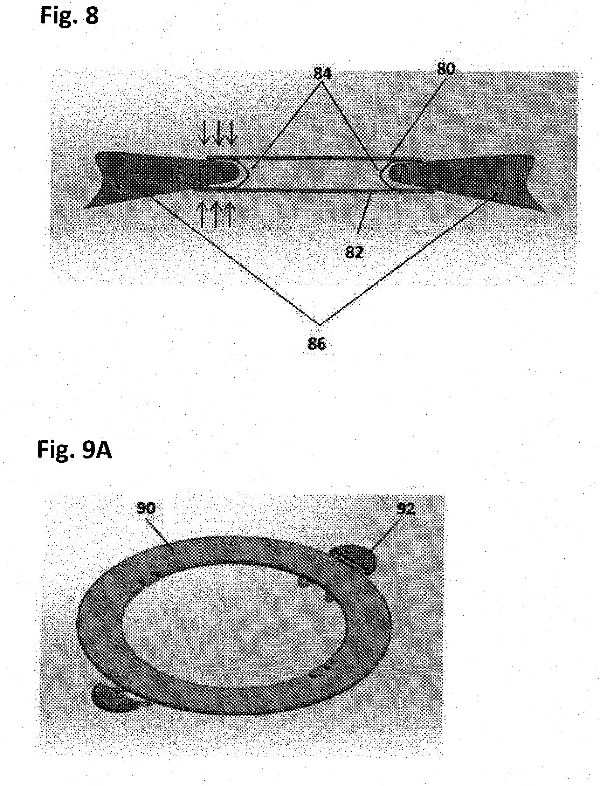

Fig. 8 depicts an alternative design of a valve support, additionally comprising a horizontally-disposed ring-shaped stabilizing element **82,** located inferior to the support element **80.** Elastic members **84** mutually connect support element **80** and said horizontal stabilizing element **82.** The annulus **86** may thus become trapped or pinched between them (as indicated by the arrows). This design may either be used without any additional stabilization elements, or in combination with any of the stabilization element embodiments described hereinabove.

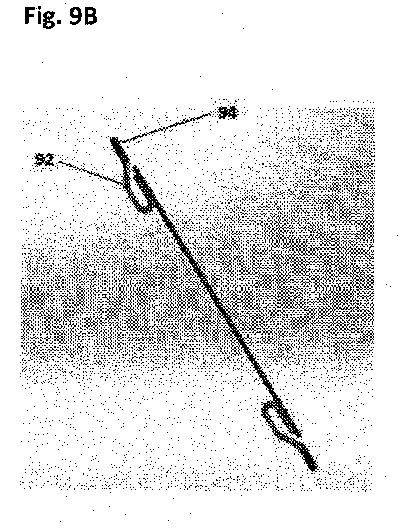

In a still further e alternative, as depicted in Fig. 9A, a valve support device as viewed from above is seen to comprise a pair of elastic stabilizing elements **92,** one on each side of the support element **90.** These stabilizing elements may be manufactured from biocompatible metals including (but not limited to) Nitinol, Cobalt and Stainless steel, and are manufactured in the form of a spring-like tab that permits the elastic forces applied by the device on the ventricular wall to be distributed over a large surface area, so as to minimize local pressure on the cardiac tissue, thus minimizing the danger of necrosis of cardiac tissue due to high-level mechanical stress. The structure of the tab-like stabilizing elements **92** may be better seen in the side view of this alternative of the device, presented in Fig. 9B. As may be seen from these figures, each tab may preferably be covered by a biocompatible fabric or mesh **94** (for example made from Dacron, PTFE etc.), the key functions of which are to assist in distributing the force, as previously explained, and also to encourage growth of cardiac tissue on the device, thus improving the attachment thereof to the heart wall. One particular advantage of using this type of stabilizing element is that it approximates the support element to the floor of the left atrium, thus essentially compressing the annulus (the stabilizing element compressing from the ventricular side and the support element compressing from the atrial side), thereby forming a "plug" that will prevent paravalvular leakage, even in cases in which the annulus is larger in diameter than the prosthetic valve, provided that the support element is larger than the annulus. In this alternative, the support element may be fitted with one or more stabilizing elements of this type, which may be distributed evenly or unevenly around the circumference of said support element. Exemplary dimensions of this tab-like stabilizing element are as follows: width 2-20 mm; and length 2 - 20mm. However, it is to be recognized that these measurements are for the purposes of illustration only, stabilizing elements of dimensions larger or smaller than these ranges being included within the scope of the present invention.

As explained hereinabove, the stabilizing elements of the present invention may be provided in the form of elongate anchoring wings, cut out of the same metallic disk used to manufacture the single-ring support device. An example of a single ring support structure of the present invention, comprising two anchoring wings of this type, is illustrated in Fig. 17. (It will be appreciated that this figure - as well as all similar figures exemplifying top views of similar devices - are intended to show said devices in their pre-crimped conformation.) The support structure **710** in this example is seen to comprise a circular support ring **712** fitted with elements **714** which permit the inner circumference of said ring to elastically deform in a radial direction (thereby facilitating the precise adaptation of the ring to a replacement valve of any size). The device also comprises two anchoring wings **716,** the basal sections **718** of which are continuous with the ring itself. Indeed, in most preferred embodiments, the wings have been cut out of the same disk as the ring itself. Finally, each of said wings also has a small aperture **719** formed close to its distal tip, the purpose of said aperture being assist the operator in gripping the support device during implementation, as will described in more detail, hereinbelow.

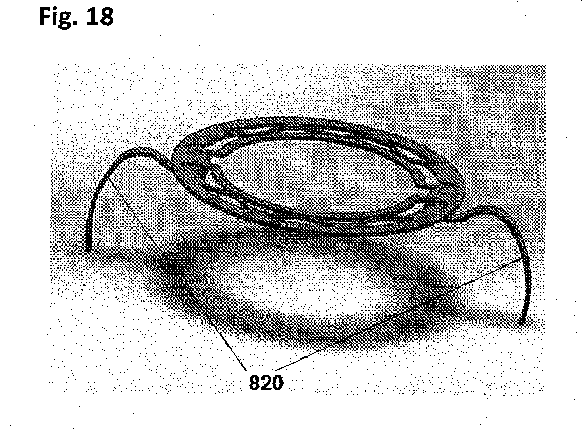

Fig. 18 shows the same valve support device following its release from the delivery catheter, and after the anchoring wings **820** have expanded into their open, working conformation.

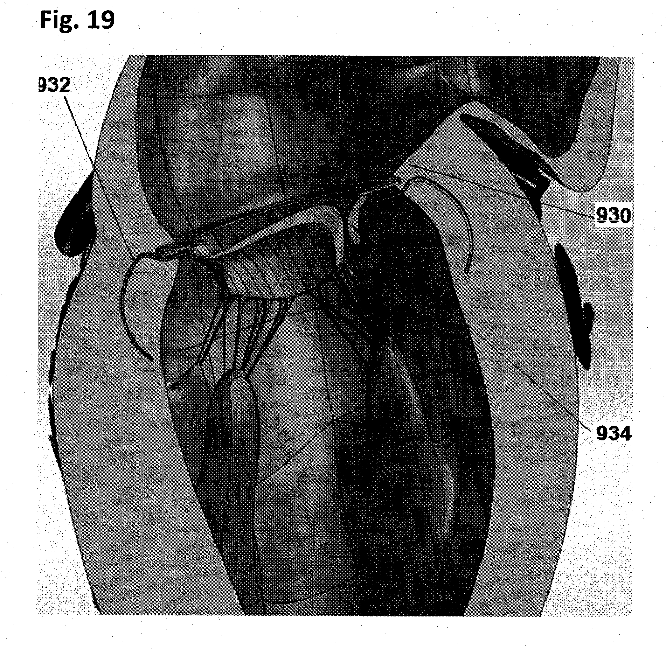

Fig. 19 illustrates the valve support device of Figs. 17 and 18 following its implantation into the heart in the region of the cardiac annulus **930.** Thus, it will be seen that the anchoring wings **932** are aligned along the commissure of native mitral valve **934,** such that the presence of the support device does not interfere with the functioning of said native valve at this stage. It is to be noted that the anchoring wings **932** compress the ventricular tissue with which they are in contact, thereby causing a slight radially-outward displacement of said tissue. (This displacement is not visible in Fig. 19, due to drawing limitations.)

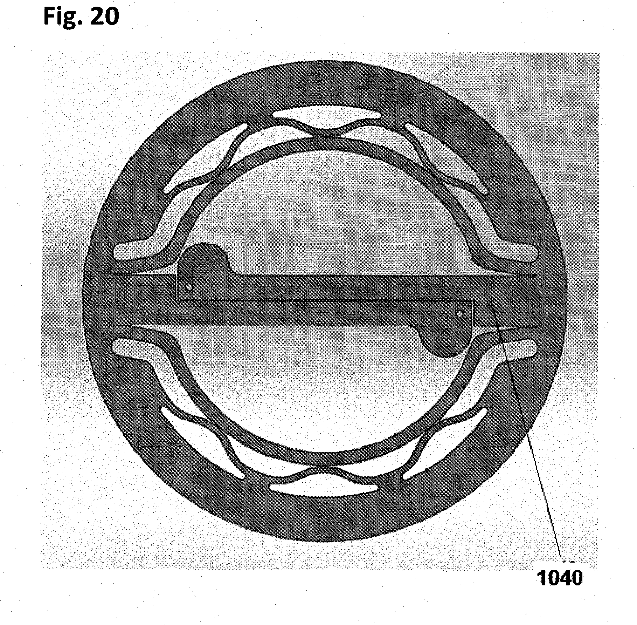

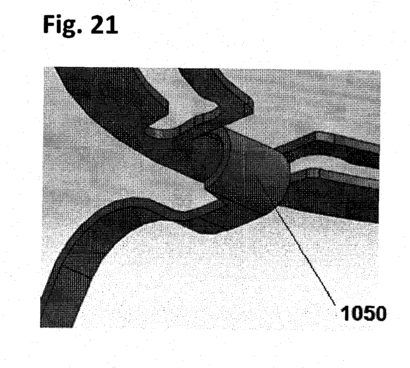

A different embodiment of this aspect of the invention is illustrated in Fig. 20, in which it may be seen that each anchoring wing has an enlarged basal section **1040.** It may be further seen in the enlarged side view of this device in its expanded conformation (shown in Fig. 21), that the expanded basal section (now shown as **1050**) contributes to the mechanical strength of the anchoring wing precisely at the point where said wing curves away from the ring support structure.

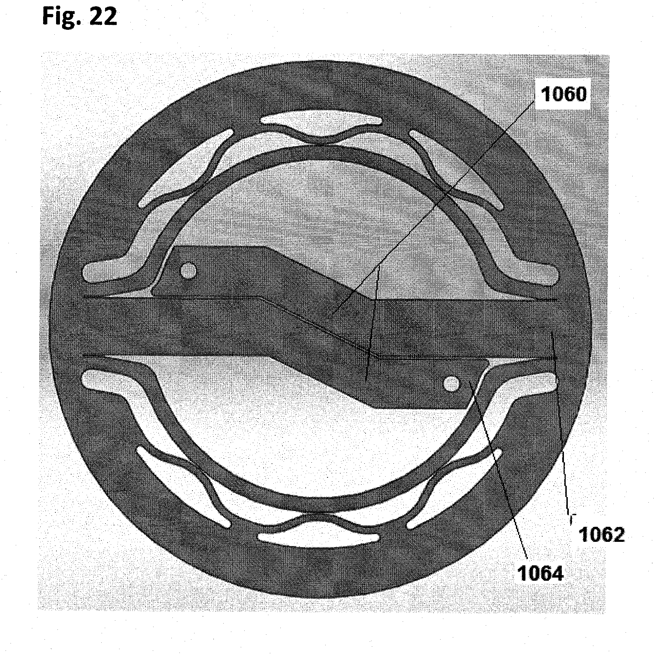

In yet another embodiment, as shown in Fig. 22, the anchoring wings **1060** are broader than the wings depicted in the earlier drawings, this increased breadth being maintained along the entire length of each of said wings, from the basal section **1062** to the distal tip **1064.** As a consequence of their greater breadth, the anchoring wings of the embodiment depicted in this figure are able to transmit a greater stabilizing force onto the ventricular tissue. This larger wing also distributes the anchoring force on a larger surface area of the heart - this is beneficial since force distribution reduces the local stress on myocardial tissue, and this may be clinically beneficial since it will prevent high stresses that may damage tissue.

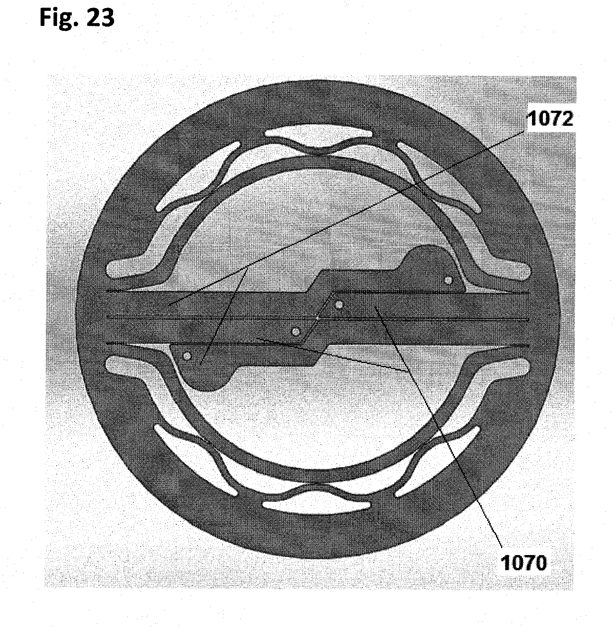

A slightly different approach is shown in Fig. 23, in which the support device comprises four anchoring wings - two short wings **1070** and two long wings **1072** which are disposed such that one short wing and one long wing are situated side-by-side on each side of the device. One advantage of this embodiment of the support device is that the presence of both a short wing and a longer wing on each side forms a compensatory mechanism such that in the event that one of said wings (e.g. the long wing) on each side does not make satisfactory contact with the ventricular wall, then the other one (the short wing) will be able to do so.

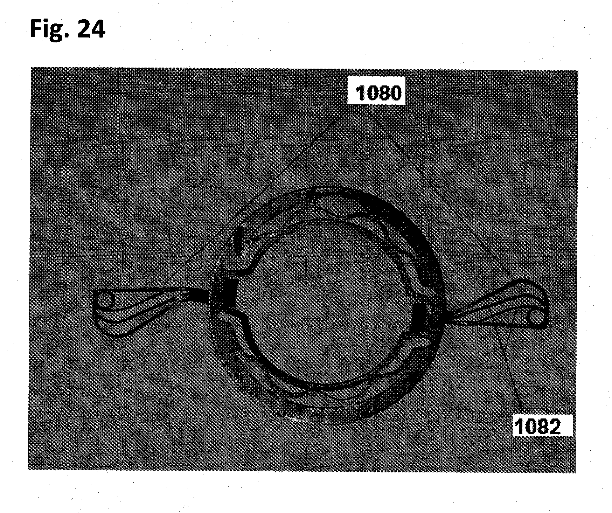

In all of the various embodiments described thus far and depicted in Figs. 17 to 23, the anchoring wings are formed as solid structures cut out of the same disk as the support ring itself. In an alternative approach, as shown in the photographic view presented in Fig. 24, the wings **1080** are constructed as open structures. This type of wing may be created, for example, by means of first cutting out a broad wing from the support ring disk, and then further removing material, such that one or more metallic strands remain within the wing. Two such strands **1082** are shown in the design depicted in Fig. 24. One advantage of this approach is that broader anchoring wings may be constructed (thereby being able to apply stabilizing forces to a larger area of the ventricular wall), without adding to the bulk or weight of said wings. As previously explained, this larger wing also distributes the anchoring force on a larger surface area of the heart - this is beneficial since force distribution reduces the local stress on myocardial tissue, and this may be clinically beneficial since it will prevent high stresses that may damage tissue.

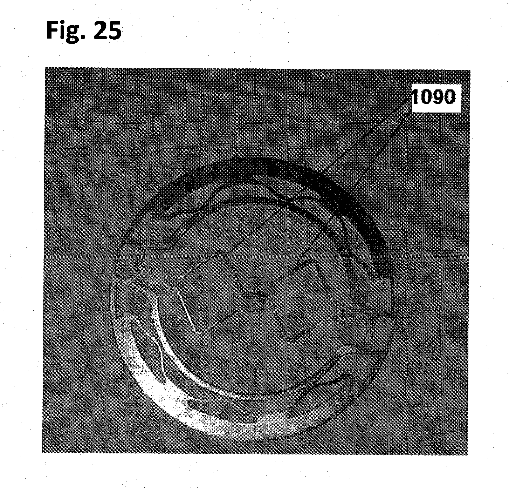

A further embodiment is shown in the photograph presented in Fig. 25. The device shown in this figure comprises wings having an open structure that are capable of existing in two different conformations - (a) an elongated, small-diameter conformation that is created during crimping during the insertion of the device into the delivery catheter and (b) a shortened, broad form, as shown in Fig. 25. As shown in the figure, the anchoring wings **1090** of this specific embodiment, in their working conformation, have a broad, diamond-like shape, and are thus capable of exerting relatively high stabilizing forces on regions of the ventricular wall close to the support device. It is to be noted that if wings having this enlarged breadth were to be formed as solid structures, it would be very difficult to crimp the device into its collapsed, delivery, conformation. Thus, the use of a skeleton structure of the type shown in this figure is highly advantageous since it combines the advantages of long, narrow wings for catheter delivery with the mechanical advantages of short, broad wings once the support device has been deployed.

The wings may be covered - either completely or, alternatively, at their distal tips only - with a fabric or other covering material. In one highly preferred embodiment, a covering material, such as biocompatible Dacron, that will permit ingrowth of cardiac tissue thereinto, is used. In this way, additional anchoring of the wings to the cardiac tissue may be achieved.

The devices may be produced by laser cutting of the Nitinol disks that are used to create the support devices. The rings are then subjected to heat treatment (at temperatures of, for example, 500 - 600 degrees C) with the wings bent in the desired working position, such that following release from the delivery device, the wings will adopt this new shape-memory position.

In some preferred embodiments, the wings will have small holes drilled through their distal-most portions, in order to allow the operator to easily grip the support device with a narrow-ended tool or wire during release from the delivery catheter, thereby facilitating the maneuvering of said device into its working position.

As disclosed hereinabove, in another group of preferred embodiments, the stabilizing elements may be provided in the form of lever-operated wings or arms. In a ***first implementation*** of the present invention, the valve support device comprises an upper, single-ring valve support device connected by means of two or more bridging elements to a lower fulcrum support ring. The valve support device in this implementation further comprises two or more anchoring arms (i.e. the same number of anchoring arms as the number of bridging elements), each of which is bent at a point along its length (as explained hereinabove) thereby defining a medial anchoring arm portion and a lateral anchoring arm portion. One end of each anchoring arm is attached to the upper (i.e. valve support) ring close to the point at which one of the bridging elements is attached. The opposite extremity of each anchoring arm is unconnected to any other structure in the device. The anchoring arms are disposed such that either the medial portion or the lateral portion thereof passes laterally through an aperture in the adjacent bridging element. Although said aperture may be formed in any convenient shape, in a preferred embodiment of this aspect of the invention, the aperture is rectangular. Either the inferior side or the superior side of said aperture acts as a fulcrum about which the anchoring arm is able to pivot.

In one preferred embodiment of this implementation of the device, the fulcrum support ring is provided in the form of a thin wire (for example, a Nitinol wire having a diameter of 0.4mm). In this embodiment, the wire "ring" is in a contracted state, and takes the form of a stirrup (rather than an open ring) prior to lateral expansion of the anchoring arms. One advantage of this contracted form is that it does not interfere with native valve leaflet function during the first step of the two-step implantation procedure. Also, the minimal surface area presented by this contracted form facilitates expansion of the stented-valve in the second step of said procedure. As the expansion of the stented-valve proceeds, the forces applied thereby onto the contracted, stirrup-shaped fulcrum support element causes said element to adopt its open ring conformation.

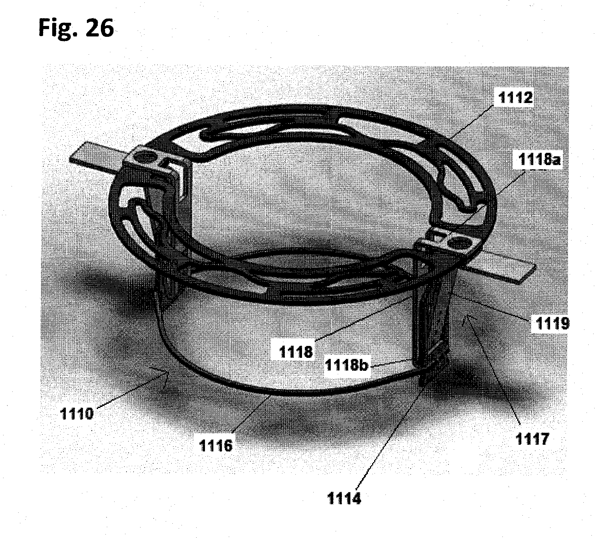

An example of this embodiment of the invention shown in its fully-expanded conformation is depicted in perspective view in Fig. 26, generally indicated as **1110,** which comprises a valve support ring **1112,** connected by two bridging elements **1114** to a lower fulcrum support ring **1116** which is constructed in the form of a thin Nitinol wire. The device comprises two anchoring arms **1117,** the medial portion **1118** of each one having an upper end **1118a** that is attached (e.g. welded) to the upper support ring, and a lower end **1118b** that ends in sharply-angled portion. The lateral portion **1119** of each anchoring arm then passes upwards and outwards from the angled portion, passing through a rectangular opening in bridging element **1114.** In the embodiment shown in this figure, the terminal portion of the distal end of lateral anchoring arm portion **1119** is angled at approximately 90 degrees to the rest of said lateral portion. However, this terminal portion may also be constructed in a variety of different forms.

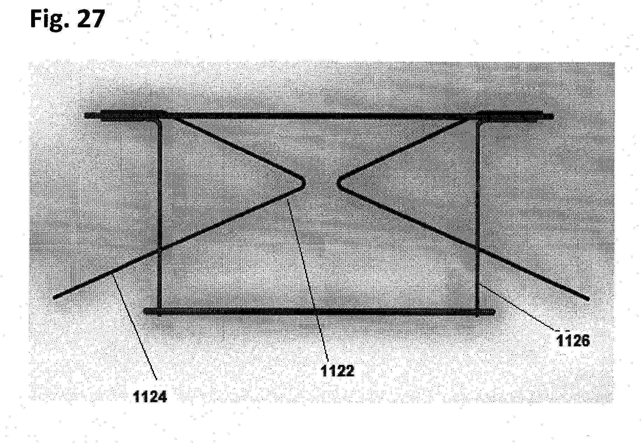

Fig. 27 provides a side-view of a device very similar to that presented in Fig. 26, but in its pre-expanded conformation. It will be seen from this figure that the angled portions **1122** of each of the two anchoring arms, are initially located close to each other, within the central space of the valve support device. Then, after implantation and expansion of the replacement valve (during the second stage of the replacement procedure), the expanding valve applies pressure to the angled portions, causing them to move laterally, while each the lateral portions **1124** of the anchoring arms pivots around its fulcrum point, which is provided by the lower edge of the rectangular opening in bridging element **1126.**

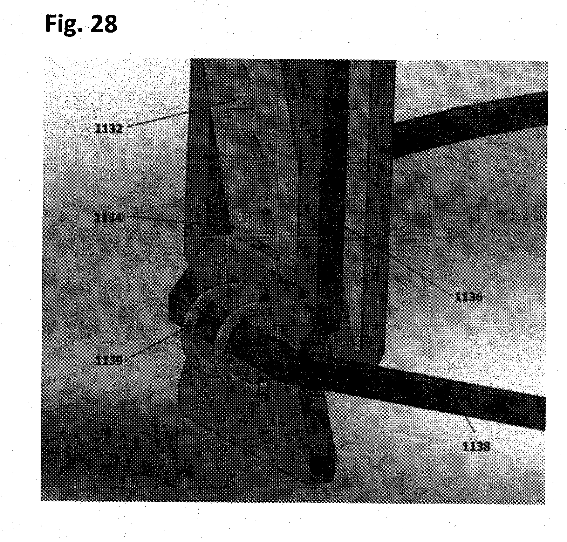

As mentioned above, in this embodiment, the lower edge of said rectangular opening acts as the fulcrum for the levered anchored arm. An enlarged view of the fulcrum point is shown in Fig. 28, in which it may be seen that the lateral portion **1132** of the anchoring arm on one side of the device is in contact with - and capable of pivoting around - the lower margin **1134** of the rectangular opening in bridging element **1136.** This figure also illustrates one way in which the bridging element **1136** may be connected to the fulcrum support ring **1138,** namely by means of small wire staples or loops **1139.**

In a ***second implementation*** of the present invention, the valve support device comprises a single-ring support element and (similar to the first implementation) further comprises two or more anchoring arms, the superior ends of which are attached to said upper support ring. In addition, the valve support device further comprises a lower ring element that is similar to the stirrup-shaped element described in connection with one of the preferred embodiments of the first implementation, hereinabove. However, in contradistinction to the first implementation, the presently-described implementation does not comprise bridging elements connecting said stirrup-shaped element to the upper support ring. Rather, each stirrup-shaped element is connected directly to each of the anchoring arms.

Functionally, this implementation differs significantly from the first implementation described above, since when the support device is in its rest position (i.e. before radial expansion) there is no fulcrum about which the levered anchoring arms are able to rotate. Rather, the fulcrum is created only after the stirrup-shaped wire is expanded (by means of the pressure applied by the expanding stented replacement valve). At a certain point, the lower wire element becomes ring-shaped. At this point, the lower wire element is unable to expand any further, and the point of attachment of each anchoring arm to the lower wire element now functions as a fulcrum, about which said anchoring arms rotate in response to the radially-outward force generated by the expanding replacement valve. It may thus be appreciated that while in the first implementation (described above), the fulcrum is present at all stages (from pre-expansion to full expansion), there is no fulcrum in the second implementation until the lower wire element has been fully expanded into its ring conformation.

In one preferred embodiment of this implementation, the device comprises two anchoring arms which are attached to the support ring (and to the lower wire element) at points separated by approximately 180 degrees from each other (as measured along the circumference of the upper support ring). In this embodiment, the valve support device is intended for implantation into the mitral valve annulus such that the anchoring arms are disposed along the valve commissure such that they do not interfere with native valve leaflet function during the first stage of the two-stage implantation procedure. In addition, the lateral portions of said anchoring arms are shaped such that they may be used to apply axially-directed forces on the ventricular wall (as described above, in relation to the first implementation of the device).

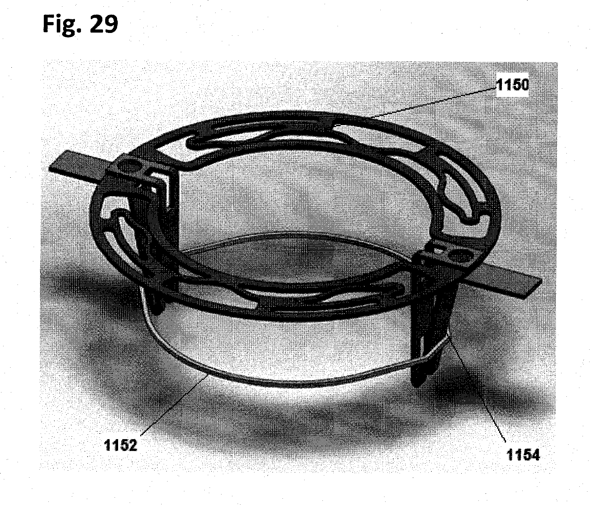

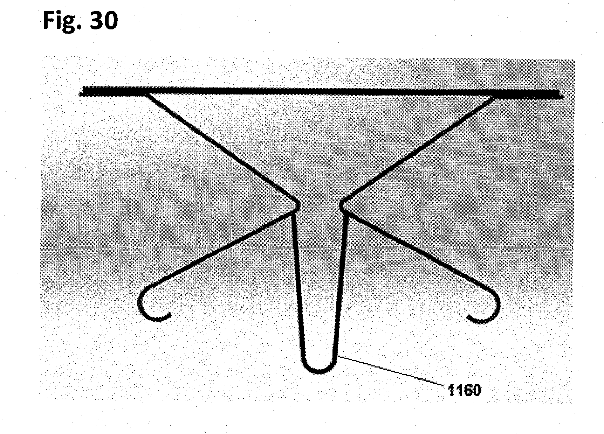

An example of a device of this type is shown in Fig. 29, which provides a perspective view of the device in its fully expanded position. As explained above, the device comprises a valve support ring **1150** and a lower fulcrum support ring **1152,** which, in its pre-expanded conformation has a stirrup-like shape (see Fig. 30). The anchoring arms **1154** are immovably attached to said support ring (e.g. by means of welding) and pivotably attached to lower ring/stirrup **1152** by, for example, small rings or staples (not shown for clarity).

A device of this implementation, similar to that illustrated in in Fig. 29, is shown in its pre-expanded conformation in side view in Fig. 30. It may be seen from this drawing that the lower fulcrum support ring **1160** is, in this conformation, stirrup-shaped and is very compact, thereby offering no resistance or interference to native valve function.

In another preferred embodiment of this implementation, the device comprises two or more anchoring arms which are constructed such that when they are in their laterally-expanded position, the angle between the medial and lateral portions of said arms is very small, such that said portions are almost in mutual contact. The small space between these portions may then be exploited in order to 'pinch' the native valve leaflets, thereby maintaining them in a fully-displaced, fully-open disposition. It may be appreciated that in this embodiment, anchoring and stabilizing of the support device is achieved by virtue of the fact that the anchoring arms firmly grip the valve leaflets which are in turn anchored to the ventricular wall tissues by means of the chordae tendineae and underlying papillary muscles. In one particular version of this embodiment, the leaflet-pinching effect exerted by the anchoring arms may be enhanced by the use of multiple prongs fitted to the inner surface of one of the portions (medial or lateral) of the anchoring arm, which are capable of penetrating the tissue of the entrapped valve leaflets upon lateral expansion of said arm, and becoming locked into correspondingly located and sized apertures on the inner surface of the other portion thereof.

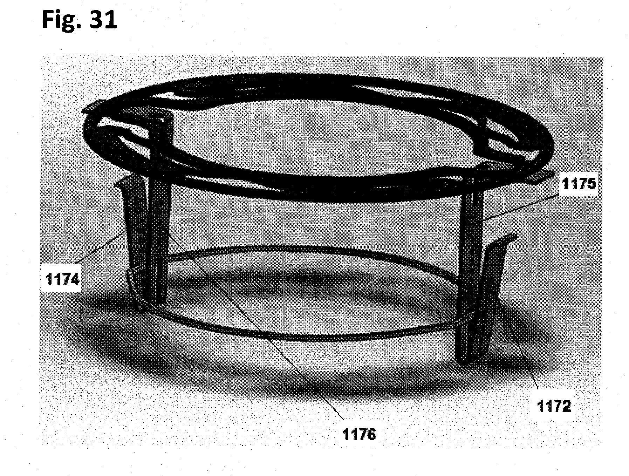

An example of this embodiment of the second implementation of the invention is shown, in perspective view, in Fig. 31. As explained hereinabove, the inner surface of one of the portions of each of the anchoring arms - in this case the lateral portion **1172** is fitted with a plurality of sharp prongs **1174.** The medial portion **1175** of each anchoring arm in this particular embodiment comprises a set of small apertures **1176** which correspond in position and size with said prongs **1174.** In use, following expansion of the replacement valve (in the second step of the two-step replacement procedure), the lateral portion **1172** of each of the anchoring arms is manipulated such that one of the native valve leaflets is trapped or 'pinched' between it and the medial portion **1175** of the same anchoring arm, and firmly held in place by prongs **1174** which penetrate the leaflet tissue and become anchored within apertures **1176.**

This implementation of the device of the invention thus possesses, *inter alia*, the following advantages:

The absence of bridging elements leads to a valve support structure that contains less material, and is therefore cheaper to construct, causes less interference with the native valve function and results in easier crimping of the device during its insertion into the delivery catheter.

The absence of bridging elements is further advantageous since there is now no need to align the anchoring arms (which in the first implementation were attached to said bridging elements) such that they are located along the valve commissure. Rather, the anchoring arms may (in one embodiment) be aligned such that each of the native valve leaflets becomes 'pinched' by the medial and lateral portions of one of the anchoring arms.

The fulcrum is created precisely when the lever effect is most needed - that is, at the point when the expanding replacement valve has caused maximum lateral displacement of the native mitral valve leaflets.

In the first two implementations of the device disclosed and described hereinabove, the support device becomes anchored to the ventricular wall only during and after expansion of the stented replacement valve. In a ***third implementation*** of the present invention, however, the valve support device comprises anchoring arms which are capable of applying both weak forces to the ventricular wall during the first stage of the two-step implantation procedure and then stronger forces during the second stage of said procedure. In order to achieve this technical effect, the device, in this implementation comprises a single-ring valve support element to which are attached two or more curved anchoring arms that are essentially devoid of straight portions. In one preferred embodiment of this implementation, said curved anchoring arms initially curve in an inferio-medial direction (i.e. towards the center of the internal space of the support ring. Then, the direction of the curvature of said arms changes such that they curve inferio-laterally, laterally, superio-laterally and then in a superior direction, finally ending in a short portion that curves back in a medial and inferio-medial direction. In this particular embodiment, the curved anchoring arm has an outline form similar to an uppercase 'D' letter, with the flatten portion of the 'D' being represented by the upper portions of said arm. During the first stage of the implantation procedure, the curved arms are capable of applying relatively weak stabilizing forces to both the lateral wall of the ventricular cavity, as well as the tissue forming the roof of said cavity. Then, during and following expansion of the stented replacement valve within the central cavity of the support ring, the curved arms of said ring are pushed outwards and (as result of their curvature) upwards, such that said arms are capable of exerting much stronger forces on the lateral and superior walls of the left ventricle. In addition, the outward and upward movement of the arms changes the angle that the terminal, free portion thereof, makes with the ventricular roof, such that the forces exerted on the ventricular tissue are along the axial direction of said terminal portion (thereby preventing the buckling of the anchoring arm which may otherwise occur if the anchoring arm would meet the ventricular roof at 90 degrees to said axial direction).

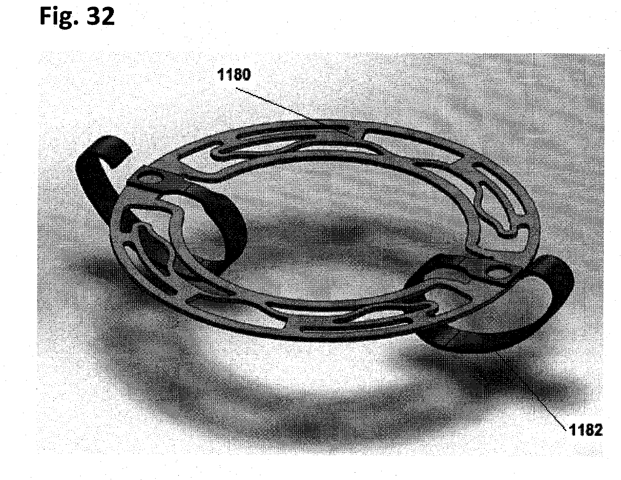

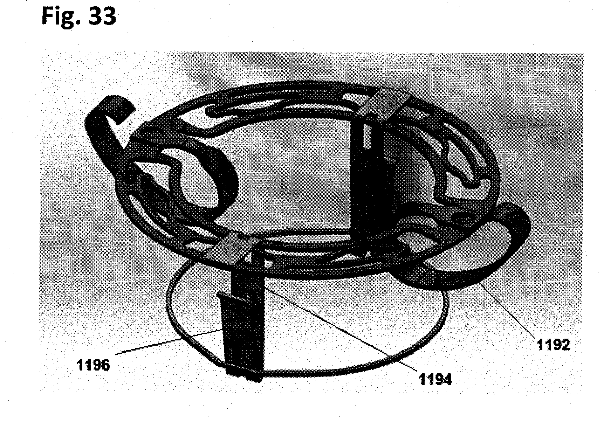

An example of this implementation of the present invention is depicted in Fig. 32. The medial ends of the curved anchoring arms **1182** are attached to the support ring **1180,** while the lateral ends of said arms are seen to curve outwards and upwards. The device depicted in this figure is in its expanded state (i.e. following expansion of the replacement valve which would be placed within the central cavity of the support device), and the lateral ends of anchoring arms **1182** are shown as if they are in a plane above the plane of support ring **1180.** However, in reality, said lateral ends would in fact come to rest in approximately the same plane as the support ring, and would apply strong stabilizing forces to the tissues of the ventricular roof. Fig. 33 shows the same implementation after

It should be noted that the third implementation of the device of the present invention does not utilize levers in order to obtain a force amplification effect.

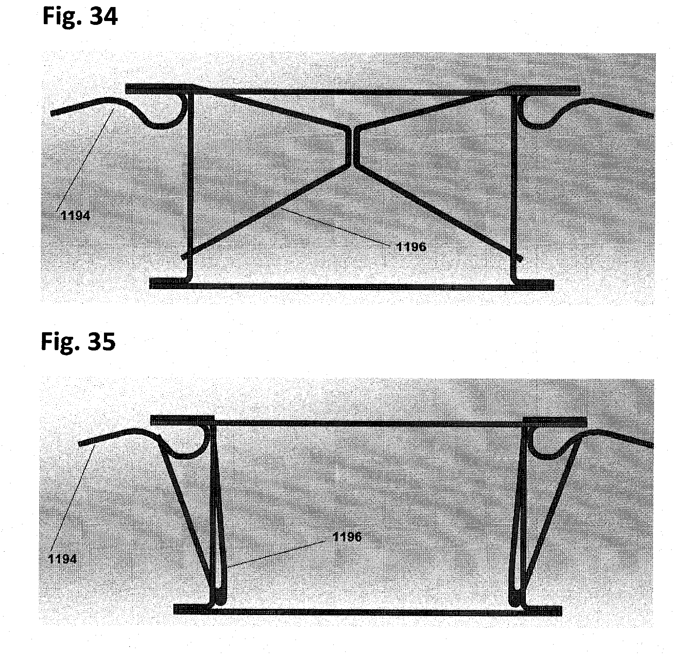

As an alternative to the third implementation of the present invention, it is also possible to construct a device comprising a combination of the first or second implementations with shorter, static anchoring arms. In such a device, the fixed arms will be used to apply relatively weak forces to the ventricular wall during the first stage of the two-stage implantation procedure, while the longer levered anchoring arms will be used to apply the stronger stabilizing forces that are required during the second stage of the procedure. In other embodiments comprising a combination of the first or second implementations with short static arms, the various anchoring elements may be arranged such that the levered anchoring arms (first or second implementations) contact the static arms (rather than ventricular tissue) during the replacement valve expansion step, thereby applying their strong stabilizing forces indirectly to the ventricular wall, that is, *via* the short static arms. An example of this embodiment can be seen in Figures 34 and 35, wherein Fig. 34 illustrates the device of the invention prior to expansion of replacement valve and Fig. 35 illustrates the device after the expansion of the replacement valve. In both figures the static arms are shown as **1194** and the levered arms are shown as **1196.**

In certain other embodiments, the device may also comprise a combination of the anchoring mechanisms of several different of the above-described implementations, for example, the curved anchoring arms of the third implementation, together with the 'leaflet pinching' embodiment of the second implementation. Such an embodiment is shown in its expanded conformation, in perspective view, in Fig. 33. In use, the pair of curved anchoring arms **1192** will be placed along the commissural line of the native mitral valve, while the medial **1194** and lateral **1196** portions of the levered anchoring arms will be in a position such that they can be used to entrap the native mitral valve leaflets therebetween.

In certain other embodiments of the first and second implementations of the present invention, the anchoring arms and/or bridging elements (first implementation) may additionally comprise a mechanism for locking the anchoring arms in their laterally-expanded position, such that they do not apply medially-directed forces on the replacement valve. In such an embodiment, the locking mechanism may be provided by a pin connected to the bridging element, said pin being capable of interacting with an appropriately-sized aperture formed within the levered anchoring arm.

All of the components of the various embodiments of the device fitted with lever-operated stabilizing arms disclosed and described hereinabove may be constructed using any suitable biocompatible material possessing shape memory and/or superelastic properties. These properties are required in order to permit the valve support device of the invention to be transformed between a collapsed conformation (such that said device may be loaded into a delivery catheter) and an expanded, working conformation. While a preferred material for use in constructing the device is Nitinol, other suitable metallic and non-metallic materials may also be used and are included within the scope of the present invention. The various embodiments described herein may be constructed using any of the standard manufacturing techniques known to the skilled artisan in this field, including laser cutting, spot welding and so on.

In some embodiments of the present invention, the careful selection of a correctly-sized valve support device will permit said support device to be self retaining in the region of the annulus following self-expansion during device delivery, as will be described hereinbelow. In other cases, however, the valve support device of the present invention will further comprise one or more heart tissue anchoring means or mechanisms connected to the support element for firmly anchoring said valve support to the cardiac tissue. In one embodiment of this aspect, the cardiac anchoring means comprise a plurality of spiral or hook-like anchors. An example of this type of anchoring means is illustrated in Fig. 1, which shows a guide catheter **16** being used to deliver a valve support device **10** of the present invention. At the stage of the delivery process shown in this figure, the support element **12** has self-expanded into their working conformations. It will be seen that the support element is fitted with two spiral cardiac attachment anchors **18,** the sharp free ends of which face laterally. The bases (i.e. medial ends) of the anchors are connected to control wires **19** that pass upwards and proximally through guide catheter **16,** eventually leaving the patient's body and ending at a proximal control console. Once the valve support device has been manipulated into the desired position (as shown in the figure), the spiral anchors **18** are caused to rotate by means of the operator manipulating the proximal ends of the control wires, thereby becoming inserted within the cardiac tissue and thus firmly anchoring the valve support device in its operating position.

It is to be noted that Fig. 1 presents only one exemplary design for the cardiac tissue anchors, and many others are possible and included within the scope of the present invention. Thus, in another embodiment, hook-like anchors are attached at various points along the surface of the valve support device. This aspect is illustrated in Fig. 10 which depicts a typical valve support device **110,** comprising a support element **120** on the surface of which are distributed a number of hook-like anchors **140.** (Four such anchors are shown in the figure.)

In some situations, it is advantageous for the cardiac tissue anchors to adopt a closed, inactive conformation during insertion of the valve support device into the body, in order to avoid both trauma to the patients tissues and to avoid premature anchoring (for example at an incorrect location). Then, when said device is correctly positioned, the anchors would be caused to move from their closed, inactive conformation to an open active position. There are a number of ways to implement this aspect. Thus, in a first implementation, the cardiac attachment anchor is constructed with two or more backwardly-pointing self-opening distal arms. During insertion and implantation, the distal arms are retained in a closed conformation by means of a small loop of resorbable suture material. Then, after a certain period of time following insertion of said attachment means into the ventricular tissue (e.g. between a few hours and a few weeks), said suture dissolves, thereby permitting the distal arms to adopt their open conformation. This aspect is illustrated in Figs. 11a and 11b: in Fig. 11a, the distal anchor arms **160** are shown retained in their closed position by means of suture **180.** In Fig. 1 lb, the required length of time has elapsed (following insertion) and the suture has dissolved, releasing the distal anchor arms and allowing them to spread apart within the cardiac tissue, thereby increasing the resistance to withdrawal offered by said anchor.

Alternatively, the anchor hooks are manufactured from a shape memory material, such as biocompatible nickel-titanium alloys (e.g. Nitinol). During insertion, the anchors are in their closed conformation, but following the implantation procedure the rise in temperature experienced during insertion into the patient's body results in opening of the anchors, as they regain their initial shape.

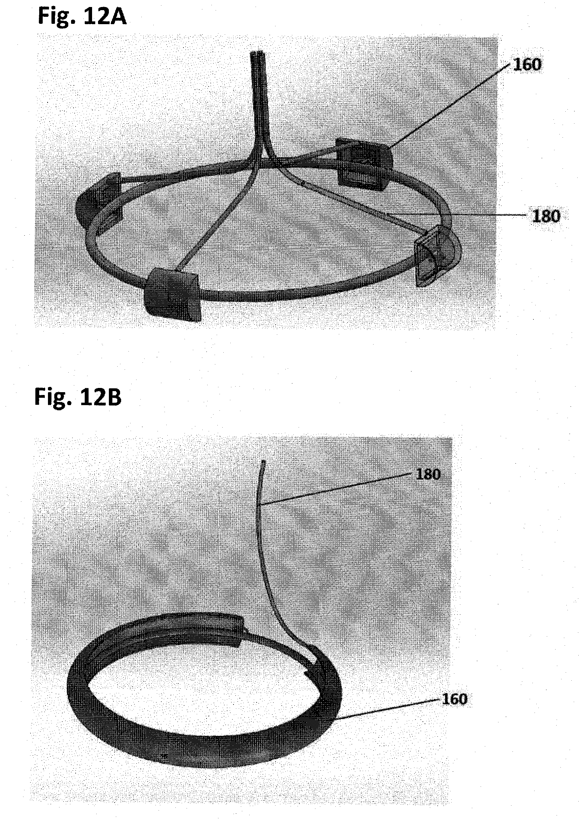

In a still further alternative, as shown in Figs. 12A and 12B, the anchor hooks are protected by a cover element **160** (such as a sleeve or a piece of tubing) which is manufactured from a material with limited flexibility, such as PET, nylon and similar biocompatible plastics. After the operator is satisfied that the valve support device has been implanted at the correct site, control elements **180** (e.g. wired) attached to the cover elements are pulled, thereby withdrawing them through the guide catheter, thus permitting the anchor hooks to freely adopt their open conformation and to become inserted into the cardiac tissue. In the design shown in Fig. 12A, each anchor is protected by its own individual cover, while in Fig. 12B a single cover element protects all of the anchors (not shown) that are attached to the support element.

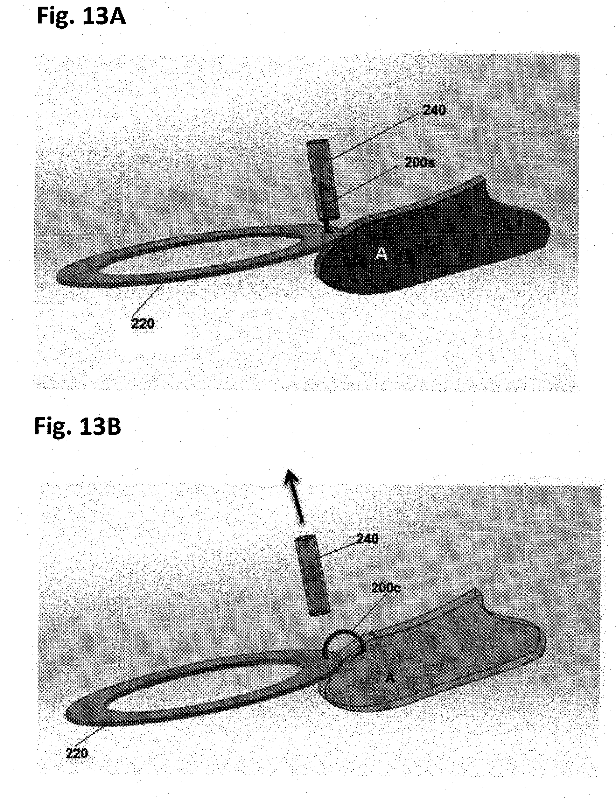

Figs. 13A and 13B illustrate a yet further alternative of this aspect. Thus Fig. 13A shows a barbed anchor **200s** attached to a support element **220** is maintained in an inactive, straight conformation by means of an overtube **240,** which also serves to protect the patient's tissues from trauma during insertion and implantation of the valve support device. Following implantation at the desired site, as shown in Fig. 20B, overtube **240** is pulled away from the anchor **200c** (for example, by means of pulling a control wire), which now adopts its "natural", curved conformation, during which shape transition, said anchor now pierces the cardiac tissue (indicated by the letter A in the figure). Suitable anchors for use in this alternative can be manufactured from shape-memory materials or from super-elasticity materials such as Nitinol, cobalt base alloy and spring-tempered stainless steel. Typically, anchors of this type will have a mid-length diameter of between about 0.2 mm and 1 mm, and a length in the range of about 2 to about 10 mm. Suitable overtubes may be manufactured from biocompatible polymers such as braided nylon and PET to a tolerance that permits a tight fit over the anchor.

It is to be noted that the cardiac tissue anchors described hereinabove may, in certain cases, be used to attach the valve support device of the present invention to the anatomical valve leaflets and chordae (in addition to, or instead of attaching said device to the inner ventricular wall). In this regard, the present invention also encompasses additional types of cardiac tissue anchor which are characterized by having a plurality of anchoring wires that advantageously become entangled within the valve leaflets and chordae. Anchors of this type are particularly suitable for use in attaching the support element to the aforementioned anatomical structures.

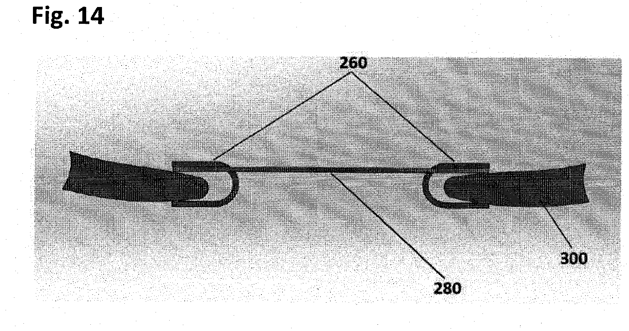

In one still further alternative, the cardiac tissue anchors may be provided in the form of small clips (similar to vascular clips used to close blood vessels during surgical procedures, and well known to the skilled artisan). An example of the use of this alternative is shown in Fig. 14, in which clip **260** is used to attach the support element **280** to the annulus **300.** Clips of this type may also be used to attach the support element to atrial wall tissue and/or anatomical valve leaflets. In one particularly preferred alternative, the clip is caused to attach to the tissue in the area of the trigone - an anatomical area, on two opposite sides of the mitral valve, which has more fibrous tissue -and which is therefore able to provide a firm base for anchoring the valve support device.

In another embodiment (not shown), the clip may be an integral part of the support element. This may be achieved by attaching one of the jaws of the clip to the valve support device, while the second of the jaws is free to be plastically deformed and to become anchored to the tissue.

In the case of certain replacement valves that may be used in conjunction with the valve support device of the present invention, the radially-outward forces exerted by the expanded replacement valve are sufficient to stably retain said valve within the inner cavity of said valve support device. However, in some instances - particularly when self-expanding replacement valves are being implanted - the radial force exerted by the expanded valve may be insufficient to ensure that it can withstand all of the physiological forces exerted therein during all stages of the cardiac cycle. In such circumstances, the single-ring support element of the valve support device may further comprise a valve engagement portion. In one embodiment, said valve engagement means comprise either inward facing or outward facing anchors, whose purpose is engage with the external struts of the replacement valve, thereby stabilizing said valve within the support device.

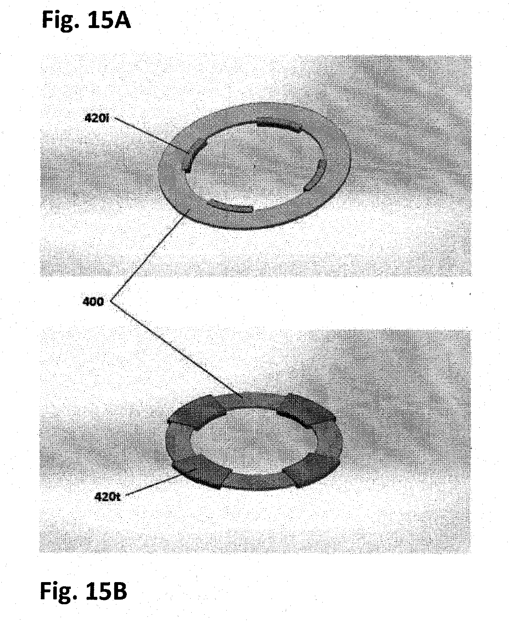

Figs. 15A and 15B show a further example of the valve engagement means, attached to an illustrative support element **400.** Thus, in Fig. 15A, four short lengths of a soft biocompatible material (such as a biocompatible fabric, silicon, PET etc.) **420i** are attached to the inner surface of support element **400.** Upon expansion of the replacement valve stent within the inner space of the valve support device, the soft material is caused to penetrate between the valve stent struts, thereby forming engagement "teeth" that serve to stabilize the replacement valve - support device assembly. Fig. 15B depicts a very similar set of four valve engagement means **420t** formed from a soft biocompatible material. However, in the case of this version, the soft material is provided in the form of tubular sleeves surrounding (partially or completely) support element **400** at the four locations shown in the figure.

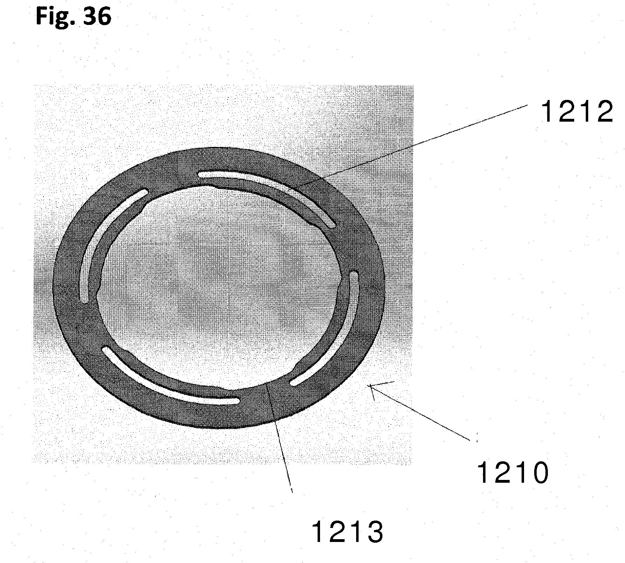

As explained hereinabove, the inner perimeter of the annular single-ring support element is able to elastically deform in a radial direction, in order to enhance the stability of the replacement valve within the central space of the valve support device. Thus, in the preferred embodiment illustrated in Fig. 36 the support element **1210** includes cut-out areas **1212,** which are cut out from the central area of the element (from the "body" of the ring), and cut-out areas **1213** which are cut out from the inner part of the element (from the internal perimeter of the ring). In this example, there are four such **1212** cut-out areas and 4 such **1213** cut-out areas. The number and shape of these cut-out areas is exemplary only, and any number and shapes may be used. An exemplary material for manufacturing the support element is biocompatible metal or alloy (for example Nitinol or stainless steel). The goal of both cut out areas **1212** and **1213** is to make the support element elastically deformable at the inner perimeter of the ring, to enable radially inward forces to be applied when a stented valve is expanded within the support element.

Exemplary sizes for the device of the invention: for example, the internal diameter of the support element **1210** in a "resting" state (the baseline stent, after the ring is deployed in the Mitral annulus, but before a stented valve is deployed and expanded within the ring) may be 25mm. An exemplary 26 mm diameter valve (e.g. the Sapien valve manufactured by Edwards Lifesciences Inc., USA) is now expanded within the support element by means of balloon expansion to a diameter of 27mm, and immediately after expansion it has some recoil to a diameter of 26mm. Since the valve was expanded within the support element, the internal ring diameter is now (after expansion) directly approximated to the valve, so the inner diameter of the support element ring is now 26mm. Since, as said in the example, the resting diameter of the support element is 25mm, than due to the elastic ability of the support element in the design of this invention, the support element now applies a radially inward force on the valve, and thus is strongly secured to the valve and prevents the valve from dislocating. Of course, sizing may change according to the desired valve, and this is an example only.

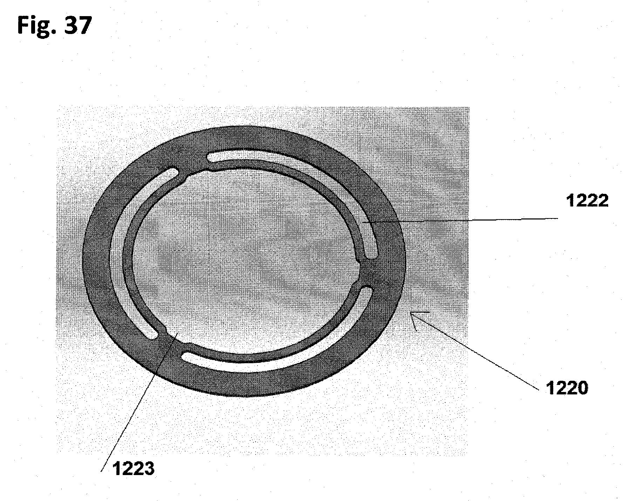

Fig. 37 illustrates another exemplary embodiment of a single-ring support element of a valve support device of the present invention In the preferred embodiment illustrated in this figure, the support element **1220** includes three cut-out areas **1222,** which are cut out from the central area of the element (from the "body" of the ring), and three cut-out areas **1223** which are cut out from the inner part of the element (from the internal perimeter of the ring). The number and shape of these cut-out areas is exemplary only, and any number and shapes may be used.

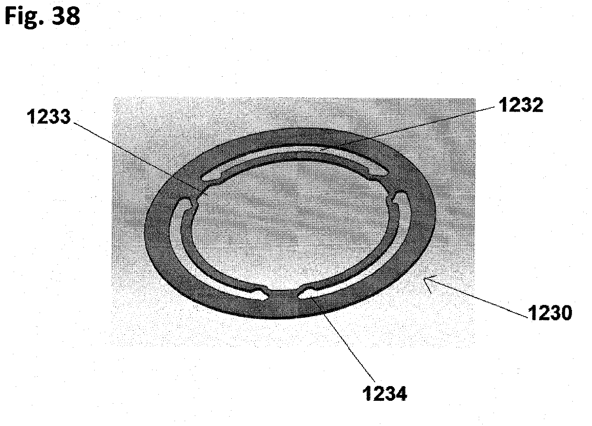

Fig. 38 illustrates a further exemplary embodiment of a single-ring support element of the present invention. In the preferred embodiment illustrated in this figure, the support element **1230** includes three cut-out areas **1232,** which are cut out from the central area of the element (from the "body" of the ring), and three cut-out areas **1233** which are cut out from the inner part of the element (from the internal perimeter of the ring). The number and shape of these cut-out areas is exemplary only, and any number and shapes may be used. In this embodiment, each cut-out area **1232** includes additional cut out areas **1234** which modify and increase the elasticity of the support element. Any number or shapes of such additional cut-out areas are within the scope of the present invention.

As explained hereinabove, in certain preferred embodiments of the present invention, the single-ring support device comprises reduced-diameter, cut-out regions in its external perimeter, the purpose of which is to act as a pressure-release element, thereby permitting the controlled, limited regurgitation of the cardiac valve, as a means of reducing the overall fluid pressure exerted on the cardiac valve support device and prosthetic valve. In this way, the stability of the implanted prosthetic valve may be improved. Thus it may be appreciated that the pressure-release feature of this aspect of the invention reduces the total fluid pressure applied on the valve support - replacement valve apparatus by the contracting heart, thus reducing the upward forces applied on said apparatus. Additionally, this design reduces the afterload against which the ventricle contracts, since it allows a controlled limited regurgitation, and thus may have beneficial clinical effects on ventricular function. In this design, the shape of the single-ring support structure does not completely cover the shape of the annulus, and does not have a complete circular shape, but rather has an outline shape that imparts the following two advantages: 1 - A part of the single-ring support structure has a larger diameter than the annulus diameter (i.e. an expanded diameter segment), thus when the support structure is expanded above the annulus - the larger diameter of the shape prevents it from "falling down" across the annulus from the atrium into the left ventricle, and thus assists in maintaining the valve support in its intended location in the mitral annulus. 2 - One or more parts of the single-ring support structure have a smaller diameter than the annulus diameter (i.e. a reduced diameter segment), thus when the support structure is expanded above the annulus, there are one or more apertures that remain "open" between the atrium and the ventricle. This actually causes a leak, or essentially a controlled "MR" (Mitral Regurgitation), the magnitude of which is pre-determined by the size and number of the apertures.

Clinical theoretical explanation:
It is pertinent at this point to explain how the intentional production of a "controlled MR" may be clinically valuable for a patient who is being treated with valve-replacement to correct his pre-existing MR. Thus, patients undergoing valve replacement for MR usually suffer from grade 3 or 4 MR, which results in significant clinical symptoms thereby necessitating clinical intervention. Optimally, the goal is to replace the valve and reach zero MR (no leak). However, it is clinically acceptable to complete a procedure such that the patient remains with a small residual MR (grade 1), since it would still be significantly better than stage 4 before the procedure, and since the device of this invention allows a trans-catheter implantation instead of surgery for valve replacement, the "cost" in outcome would be stage 1 MR (with a minimally-invasive procedure) instead of zero MR (using a surgical approach), this would be clinically beneficial for some patients, especially those having co-morbidities associated with a very high surgical risk.

An additional advantage of this embodiment, in which the apertures between each of the reduced diameter segments and the adjacent portion of the annulus permit the limited peri-valvular flow of blood between the ventricle and the atrium, is that after implantation of the valve support there is maintained a "controlled" or "limited" amount of regurgitation (flow during systole from the ventricle into the atria through the perivalvular apertures). This reduces the afterload, the force against which the left ventricle (LV) contracts, and may be advantageous in cases of reduced systolic performance of the left ventricle. Such afterload reduction may potentially be beneficial to improve left ventricular performance, reduce LV wall stress and oxygen consumption.

Sizing example and explanation: The following sizes are exemplary only, and are provided in order to illustrate the principle on which this embodiment of the present invention is based.

For an exemplary mitral annulus diameter of 35mm. The inner diameter of the single-ring support element has to be appropriate for the expanded diameter of the stented replacement valve which is to be expanded in the valve support. For an exemplary Sapien 26mm valve, the inner diameter of the upper ring is approximately 26mm. The outer diameter of the support element should be larger than the annulus diameter, in order to prevent the device from "falling" into the ventricle, and in order to assist in prevention of para-valvular leak. Hence for this example an outer diameter of 37mm is chosen. However, at least one part of the support ring will have a diameter which is smaller than 35mm (for example a cut out will be made in a part of the outer perimeter of the ring, thereby reducing the local diameter to only 33mm), thus causing a small aperture between the outer edge of the upper ring and the mitral annulus. During systolic ventricular contraction these one or more apertures function as a pressure release mechanism - they release some of the pressure (upward force) applied on the valve support-valve apparatus, and thus reduce the risk that the apparatus will be dislocated out of position.

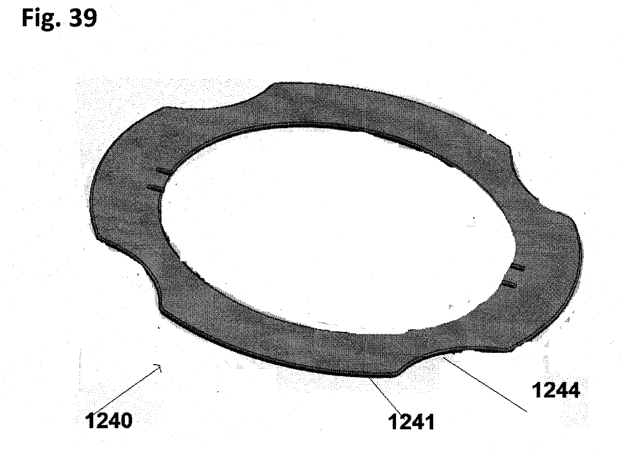

Thus, Fig. 39 provides a perspective view of an exemplary embodiment of a valve support of this embodiment of the present invention in an expanded configuration. Valve support **1240** includes an annular single-ring support element **1241,** having four areas of reduced diameter (cut-out areas from the perimeter of the ring), **1244.** This number of such smaller diameter areas **1244,** as well as their size and shape, are given by way of example only.

In another preferred embodiment, as explained hereinabove, the annular support element is fitted with least one wing-like drape element made of a biocompatible fabric. The drape functions as a sealing element, positioned between the support element and the mitral annulus, thereby preventing paravalvular leakage after implantation of the valve support in the mitral annulus.

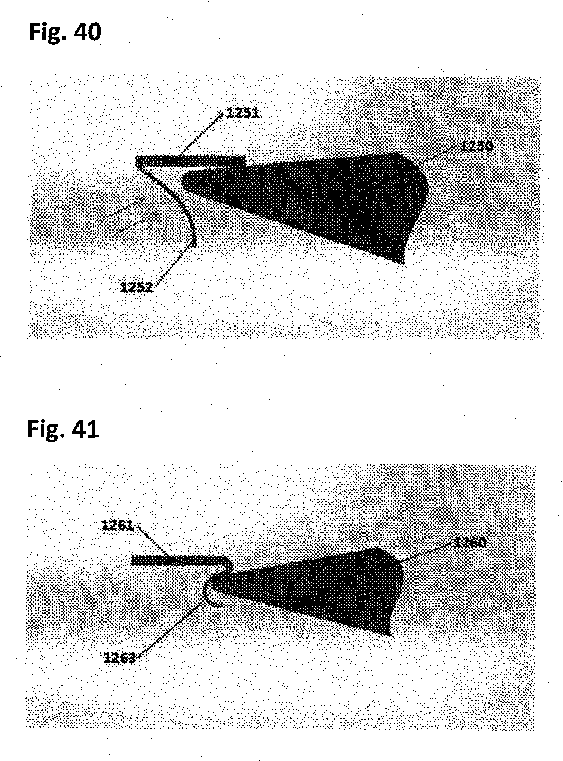

Thus, Fig. 40 illustrates a side view of an exemplary single-ring support device **1251** of the present invention, shown in position on the mitral annulus **1250,** and comprising a fabric drape **1252** attached to the inner circumference of the ring. The position of the drape on the inner circumference of the ring support presents several distinctive advantages: the drape functions as a "valve leaflet" between the mitral annulus and the upper ring, thus during systole, when fluid flows out from the ventricle, the drape is pushed upwards, towards the annulus, by the flow of blood, and this movement improves the sealing between the upper ring and the annulus, (indicated by the arrows in Fig. 40), thus essentially functioning as a valve between the ring and the annulus, and thus preventing paravalvular leakage.

Figure 41 illustrates a side view of an exemplary single-ring support device **1261** of the present invention, shown in position on the mitral annulus **1260,** with a fabric drape **1263** attached to the outer circumference of said ring. The position of the drape on the outer part of the ring support allows it to function as a sealing element between the ring and the annulus (similar to the function of a sealing "o" ring), so when the ring is approximated and attached to the area of the annulus the drape functions to seal the annulus and prevents paravalvular leakage. In some embodiments of this invention the length of the drape is such that the edge of the drape extends into the left ventricle (as shown in Fig. 41). This is advantageous since this extended drape element improves the sealing and prevents leakage between the outer area of the ring and the mitral annulus.

Exemplary materials for the drape of the invention are any kind of biocompatible fabric, for example Dacron, ePTFE. Exemplary sized of the drapes of the invention are length of 2mm - 20mm and width of 2mm-60mm, thus covering a part of the ring or the whole circumference of the ring.

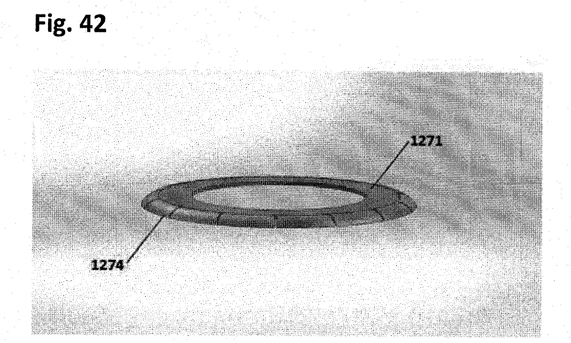

Fig. 42 illustrates a perspective view of an exemplary single-ring support device **1271** of the present invention, exemplifying multiple fabric drapes **1274.** Five such separate drapes are shown, with the rest of the drapes not shown in the illustration. Preferably there is a small overlap between drapes, such that there is no leakage between adjacent drapes.

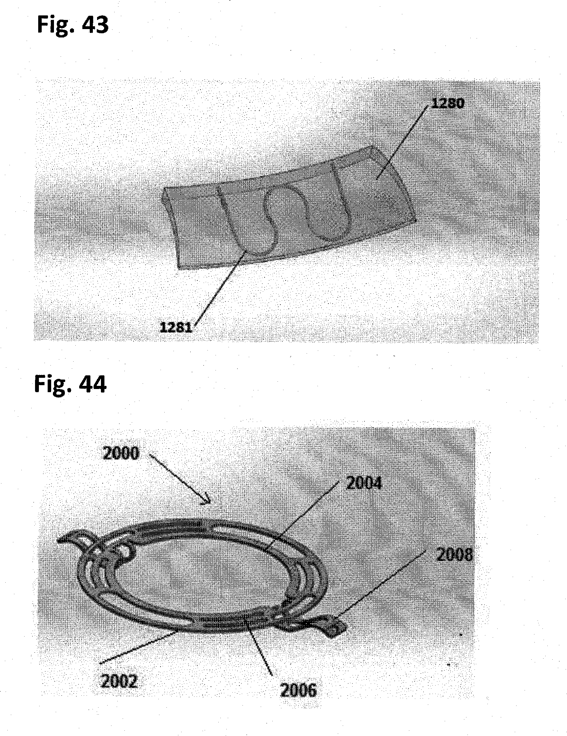

Fig. 43 illustrates a further design for the fabric of a drape of the invention. Drape **1280** as shown in this figure is made of biocompatible fabric. In order to give the drape a stable form (in order that it will have a predetermined shape), a biocompatible metal wire **1281** is sewn into the material of the drape during its manufacturing. Exemplary materials for the wire are stainless steel or Nitinol. The metal wire can be shaped according to a predetermined requirement, and is able to maintain this shape due to the mechanical properties of the wire. The advantage of this predetermined shaping is that the shape may be designed such that it will improve the sealing between the ring and the annulus, so that the flow will direct the drape towards sealing the annulus, moving the drape closer to the annulus and preventing paravalvular leakage.

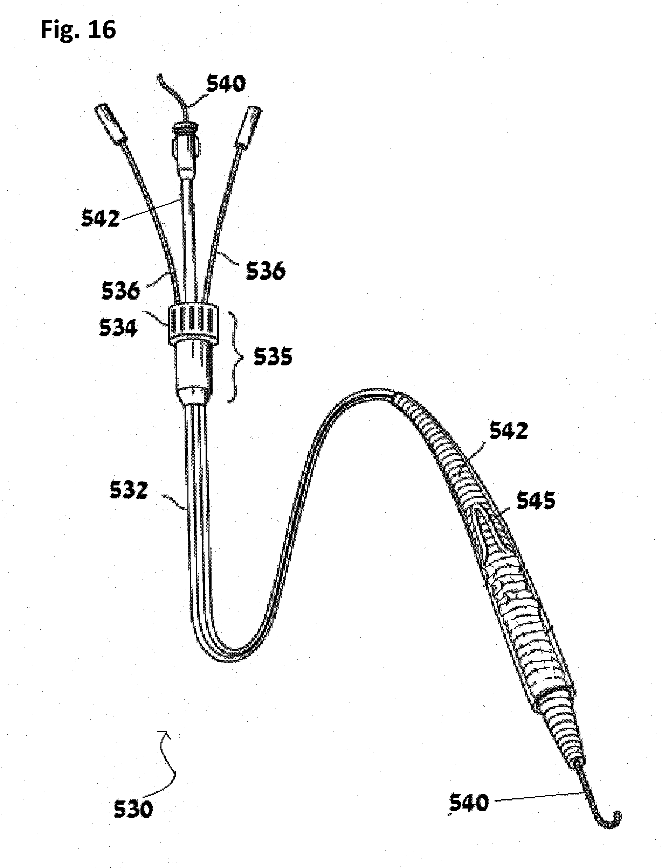

Delivery of the single-ring valve support device of the present invention is accomplished using essentially the same method and delivery device as disclosed in co-owned, co-pending US application no. 13/224,124, filed on September 1, 2011. Briefly, the method of delivery involves the use of a delivery device **530,** as illustrated in perspective view in Fig. 16. The device, as shown in this figure, comprises an actuation portion **535,** an actuator, **534,** an elongate body **532,** and a guidewire lumen **542** which is adapted to be advanced distally over guidewire **540** to advance delivery device **530** to a target location within the subject. The valve support device of the present invention in its collapsed conformation **545** is contained within the lumen of the delivery device, and is connected to coupling members **536.** The distal regions of device coupling members **536** are releasably secured to the valve support device during the deployment procedure, but are also adapted to be controllably released from said valve support device, in order to release it from the delivery device. Coupling members **536** can be actuated by actuating their proximal portions external to the patient to control movement of the valve support device.

The elongate body **532** can be, for example without limitation, a catheter, examples of which are well known. Actuation portion **535** can be, for example without limitation, a touhy borst, allowing rotation of actuator **534** to control the axial movement of elongate body **532.** Guiding lumen **542** can be, for example without limitation, a corrugated steel reinforced lumen to allow for sufficient flexibility while being advanced through the vasculature. Guiding lumen **542** can also be any other type of suitable guiding lumen.

Following release of the valve support at the desired site, it is allowed to expand in order that it may adopt its working conformation, securing itself against the lateral wall of the cardiac lumen, in the atrium above the mitral valve annulus, in the ventricle below the annulus or within the annulus itself. In some embodiments the support element includes one or more cardiac anchoring elements, such as in the form of anchors, barbs, clips, etc. and/or stabilizing elements (as described hereinabove), that help secure said support element against cardiac tissue, or that are adapted to pierce into cardiac tissue to secure the support element to cardiac tissue. One or more fixation and/or stabilizing elements, if used, can be disposed around the periphery of the support element. They can assume a collapsed, or delivery configuration for delivery of the system, but can deploy to an expanded, or anchoring, configuration, when released from the delivery system. For example, the fixation elements can be an elastic material that self-expands to an anchoring configuration. Alternatively, the fixation elements can be actuated to reconfigure them to a fixation configuration. In some embodiments, however, the one or more fixation elements are not adapted to change configurations. Further details concerning the subsequent stage of deploying and expanding a commercially-available replacement valve (of any type suitable for the procedure in question as determined by the clinician) are disclosed in co-pending US application no. 13/224,124, filed on September 1, 2011.

Access to the mitral valve or other atrioventricular valve will preferably be accomplished through the patient's vasculature percutaneously (access through the skin). Percutaneous access to a remote vasculature location is well-known in the art. Depending on the point of vascular access, the approach to the mitral valve can be antegrade and require entry into the left atrium by crossing the interatrial septum. Alternatively, approach to the mitral valve may be retrograde where the left ventricle is entered through the aortic valve. Alternatively, the mitral valve can be accessed transapically, a procedure known in the art. Additional details of an exemplary antegrade approach through the interatrial septum and other suitable access approaches can be found in the art, such as in U.S. Patent No. 7,753,923, filed August 25, 2004.

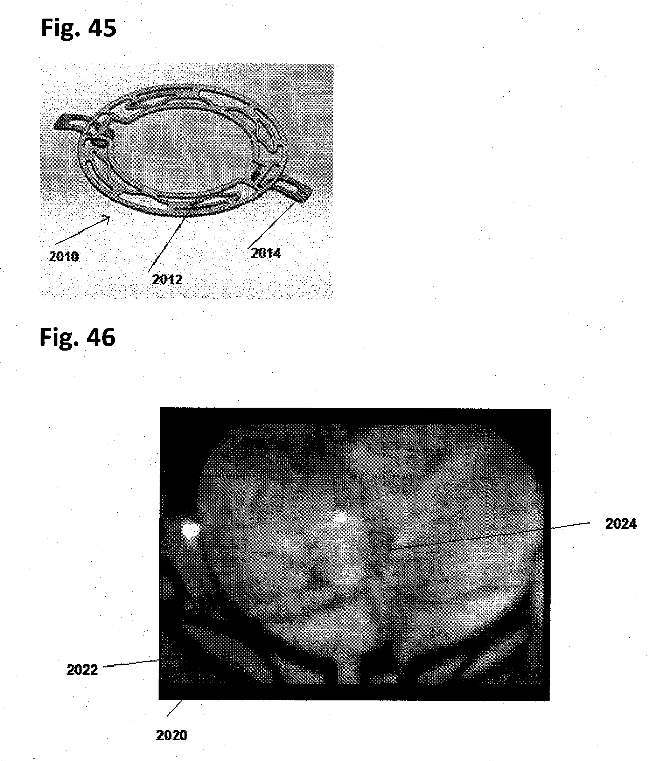

Figs. 44 and 45 depict two versions of the single-ring valve support device that were developed for use in conjunction with two different classes of prosthetic aortic valve, in order to be able to use said prosthetic valves to replace dysfunctional mitral valves. Thus, Fig. 44 shows, in perspective view, a single-ring support device **2000** for use in conjunction with a self-expanding aortic valve. It may be seen that the device has an outer perimeter **2002** and an inner perimeter **2004,** and that a number of linear slots **2006** have been created in the material of the ring between said perimeters. In this way, the inner perimeter of the ring-support has been rendered elastically deformable. The figure also shows that the support device is fitted with two short stabilizing wings **2008** positioned such that they are 180 degrees apart. The support device **2010** shown in Fig. 45 is intended for use with a balloon-expandable aortic valve, and is similar in general structure to the device in Fig. 44, having a series of slots **2012** and two short stabilizing wings **2014.** However, the slots shown in the case of this device differ both in terms of the complexity of their shape, and in the fact that they occupy a larger surface area than those shown in the previous figure. These differences result in an inner ring perimeter that has greater elasticity, a feature which is important in relation to the use of this support device in conjunction with balloon-expandable prosthetic aortic valves.

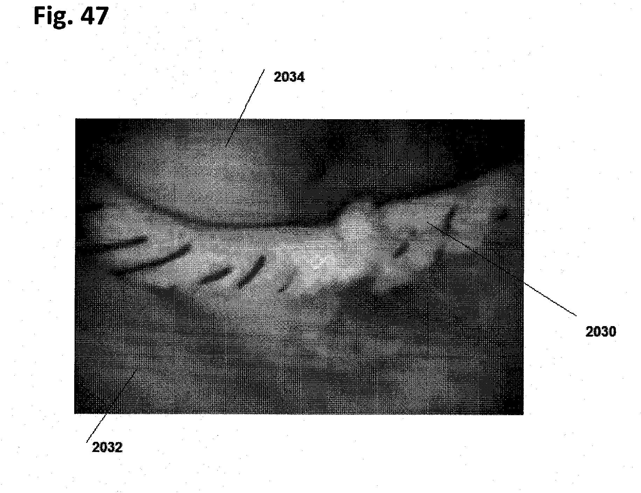

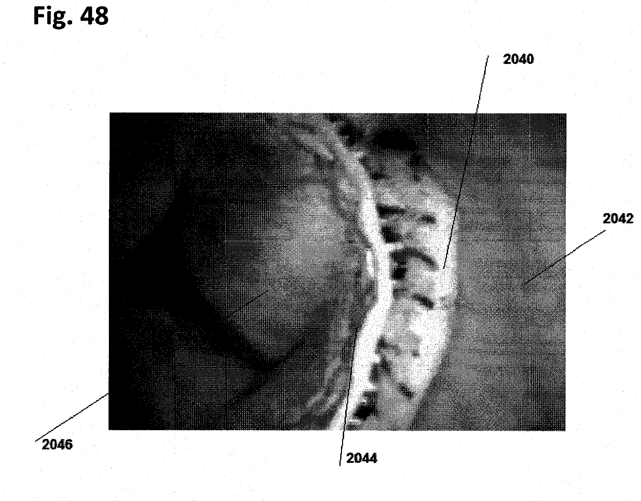

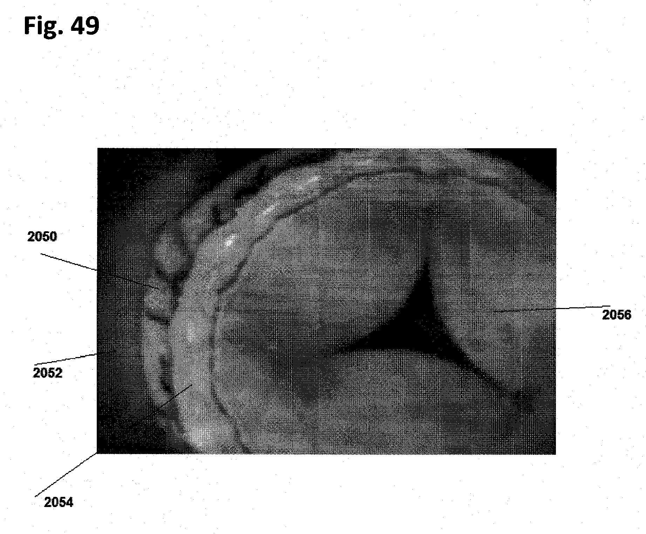

Figures 46 to 49 are photographs showing the successful implantation of single-ring valve support devices of the present invention in cadaver hearts. Thus, Fig. 46 shows a single ring support device **2020** implanted in a mitral annulus **2022** position in a cadaveric heart. The heart is connected to a pulsating pump, which provides flow and thus causes the mitral valve to open and close. It is to be noted that the device in this figure is shown without covering fabric. The photograph illustrates that when the support device is located on the annulus, it does not interfere with the closure of the native mitral valve **2024** (shown closed in the photo) - and thus maintains stable hemodynamics and allows for timely and safe deployment of a prosthetic valve within the support device in a two-stage implantation procedure as explained hereinabove. Fig. 47 presents an enlarged view of a similar valve support device **2030,** implanted above a mitral annulus **2032,** said device being, in this case, covered with a biocompatible fabric. The native mitral valve leaflets **2034** are shown in their closed position. Fig. 48 shows a similar fabric-covered valve support device **2040,** positioned above the mitral annulus **2042.** In the example shown in this figure, however, a prosthetic aortic valve **2044** has been implanted and expanded within the central space of said support device. The leaflets **2046** of the prosthetic valve are also clearly seen in this figure. Fig. 49 presents an upper view of the same prosthetic valve, having a stent portion **2054** and three leaflets **2056,** wherein said valve is firmly held in place by valve support device **2050,** which itself is shown implanted above mitral annulus **2052.** These photographic pictures of the valve support device of the present invention deployed in cadaveric hearts demonstrate that the stented valve is anchored very firmly to the single ring support device, and is not displaced, even at the high pressures generated by the pulsating valve (pressures greater than 150 mmHg, which are similar to those seen in hypertension).

While the support structures herein are generally described as a support for replacement mitral valves, they can be delivered to a desired location to support other replacement cardiac valves, such as replacement tricuspid valves, replacement pulmonic valves, and replacement aortic valves.

While some embodiments have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure.

## Claims

1. A prosthetic cardiac valve support device (10) (30) (40) (50) (60) (110) adapted for endovascular delivery to a cardiac valve, comprising a single ring-shaped support element (712, 1210, 1220, 1241, 2000) having an inner diameter (22i) and an outer diameter (22e), wherein said support element (712, 1210, 1220, 1241, 2000) has an outer perimeter (2002) that is entirely rigid, wherein said support element (712, 1210, 1220, 1241, 2000) is fitted with one or more intra-ventricular and/or intra-atrial stabilizing elements (3236, 716,), and wherein said support element (712, 1210, 1220, 1241, 2000) has a collapsed delivery configuration and a deployed configuration ***characterised in that*** said support element (712, 1210, 1220, 1241, 2000) comprises one or more cut-out areas (714, 1212-3, 1232-4, 1244, 2006), which enable the inner perimeter (2004) of the single-ring support element (712, 1210, 1220, 1241, 2000) is able to elastically deform in a radial direction.

2. The valve support device (10) (30) (40) (50) (60) (110) according to claim 1, wherein the support element (712, 1210, 1220, 1241, 2000) in its deployed configuration has the form of a flat annular ring, and wherein the difference (Rd) between the outer radius and the inner radius of said annular ring is in the range of 1-14 mm.

3. The valve support device (10) (30) (40) (50) (60) (110) according to claim 2, wherein the ratio between Rd and the thickness of the single-ring support element (712, 1210, 1220, 1241, 2000) is in the range of 10:1 and 20:1.

4. The valve support device (10) (30) (40) (50) (60) (110) according to claim 2, wherein the inner radius of the single-ring support element (712, 1210, 1220, 1241, 2000) is in the range of 23-29 mm and the outer radius thereof is in the range of 30 - 50 mm.

5. The valve support device (10) (30) (40) (50) (60) (110) according to claim 2, wherein the thickness of said support device (10) (30) (40) (50) (60) (110) is in the range of 0.25 - 0.6 mm.

6. The valve support device (10) (30) (40) (50) (60) (110) according to claim 1, wherein the stabilizing elements (32,36, 716) are selected from the group consisting of complete ring structures, partial rings, curved arms or wings, elongate arms or wings and levered arms or wings.

7. The valve support device (10) (30) (40) (50) (60) (110) according to claim 1, further comprising a wing-like drape element formed from a biocompatible fabric (94) that is attached to the single-ring support element (712, 1210, 1220, 1241, 2000).

8. The valve support device (10) (30) (40) (50) (60) (110) according to claim 1, wherein the support element (712, 1210, 1220, 1241, 2000) is fitted with replacement valve engagement means adapted to securely engage a replacement heart valve.

9. An endovascular or transapical delivery system for use in the replacement of a mitral valve (934), comprising:
a) a prosthetic cardiac valve support device (10) (30) (40) (50) (60) (110) comprising a single ring-shaped support element (712, 1210, 1220, 1241, 2000) with a collapsed delivery configuration and a deployed configuration, according to any one of the preceding claims; and
b) a replacement heart valve comprising an expandable anchor and a plurality of leaflets; wherein said replacement heart valve is adapted to be secured to said prosthetic cardiac valve support device (10) (30) (40) (50) (60) (110).

10. The system according to claim 9, wherein the replacement heart valve is a prosthetic aortic valve.

## Patentansprüche

1. Eine prothetische Herzklappen-Haltevorrichtung (10) (30) (40) (50) (60) (110), ausgelegt für die endovaskulare Versorgung einer Herzklappe, umfassend ein als Einzelring geformtes Trägerelement (712, 1210, 1220, 1241, 2000) mit einem Innendurchmesser (22i) und einem Außendurchmesser (22e), wobei das Trägerelement (712, 1210, 1220, 1241, 2000) einen Außenumfang (2002) hat, der vollständig starr ist, wobei das Trägerelement (712, 1210, 1220, 1241, 2000) mit einem oder mehreren intraventrikulären und/oder intraatrialen Stabilisierungselementen (3236, 716) ausgestattet ist, und wobei das Trägerelement (712, 1210, 1220, 1241, 2000) eine zusammengeklappte Versorgungskonfiguration und eine entfaltete Konfiguration aufweist , *die **dadurch gekennzeichnet ist, dass*** das Trägerelement (712, 1210, 1220, 1241, 2000) einen oder mehrere Aussparungsbereiche umfasst (714, 1212-3, 1232-4, 1244, 2006), die es dem Innenumfang (2004) des Einzelring-Trägerelements (712, 1210, 1220, 1241, 2000) erlauben, sich elastisch in radialer Richtung zu verformen.

2. Klappen-Haltevorrichtung (10) (30) (40) (50) (60) (110) gemäß Anspruch 1, wobei das Trägerelement (712, 1210, 1220, 1241, 2000) in seiner entfalteten Konfiguration die Form eines flachen kranzförmigen Rings hat, und wobei der Unterschied (Rd) zwischen dem Außenradius und dem Innenradius dieses kranzförmigen Rings zwischen 1-14 mm beträgt.

3. Klappen-Haltevorrichtung (10) (30) (40) (50) (60) (110) nach Anspruch 2, wobei das Verhältnis zwischen Rd und der Dicke des Einzelring-Trägerelements (712, 1210, 1220, 1241, 2000) zwischen 10:1 und 20:1 beträgt.

4. Klappen-Haltevorrichtung (10) (30) (40) (50) (60) (110) nach Anspruch 2, wobei der Innenradius des Einzelring-Trägerelements (712, 1210, 1220, 1241, 2000) bei 23-29 mm und der Außenradius zwischen 30-50 mm beträgt.

5. Klappen-Haltevorrichtung (10) (30) (40) (50) (60) (110) nach Anspruch 2, wobei die Dicke der Haltevorrichtung (10) (30) (40) (50) (60) (110) zwischen 0,25-0,6 mm beträgt.

6. Klappen-Haltevorrichtung (10) (30) (40) (50) (60) (110) nach Anspruch 1, wobei die Stabilisierungselemente (32,36, 716) ausgewählt sind aus der Gruppe, bestehend aus kompletten Ringstrukturen, Teilringen, gewölbten Armen oder Flügeln, länglichen Armen oder Flügeln und gehebelten Armen oder Flügeln.

7. Klappen-Haltevorrichtung (10) (30) (40) (50) (60) (110) nach Anspruch 1, ferner umfassend ein flügelähnliches Vorhangelement, das aus einem biologisch verträglichen Gewebe (94) hergestellt und mit dem Einzelring-Trägerelement (712, 1210, 1220, 1241, 2000) verbunden ist.

8. Klappen-Haltevorrichtung (10) (30) (40) (50) (60) (110) nach Anspruch 1, wobei das Trägerelement (712, 1210, 1220, 1241, 2000) mit Ersatzklappen-Einsetzvorrichtungen ausgestattet ist, die dafür ausgelegt sind, eine Ersatzherzklappe sicher einzusetzen.

9. Ein endovaskulares oder transapikales Versorgungssystem für die Verwendung beim Austausch einer Mitralklappe (934), umfassend:
a) eine prothetische Herzklappen-Haltevorrichtung (10) (30) (40) (50) (60) (110), die ein als Einzelring geformtes Trägerelement (712, 1210, 1220, 1241, 2000) mit einer zusammengeklappten Versorgungskonfiguration und einer entfalteten Konfiguration, nach einem der vorhergehenden Ansprüche umfasst; und
b) eine Ersatzherzklappe, die einen erweiterbaren Anker und eine Vielzahl von Segeln umfasst; wobei diese Ersatzherzklappe dafür ausgelegt ist, an der prothetischen Herzklappen-Haltevorrichtung (10) (30) (40) (50) (60) (110) befestigt zu werden.

10. System nach Anspruch 9, wobei es sich bei der Ersatzherzklappe um eine prothetische Aortenklappe handelt.

## Revendications

1. Dispositif de support de valvule cardiaque prothétique (10) (30) (40) (50) (60) (110) adapté pour pose endovasculaire à une valvule cardiaque, comprenant un élément de support à forme d'anneau unique (712, 1210, 1220, 1241, 2000) ayant un diamètre interne (22i) et un diamètre externe (22e), dans lequel ledit élément de support (712, 1210, 1220, 1241, 2000) a un périmètre externe (2002) qui est entièrement rigide, dans lequel ledit élément de support (712, 1210, 1220, 1241, 2000) est pourvu d'un ou plusieurs éléments stabilisants intraventriculaires et/ou intra-auriculaires (3236, 716), et dans lequel ledit élément de support (712, 1210, 1220, 1241, 2000) présente une configuration de pose affaissée et une configuration déployée ***caractérisée en ce que*** ledit élément de support (712, 1210, 1220, 1241, 2000) comprend une ou plusieurs zones de découpe (714, 1212-3, 1232-4, 1244, 2006), qui permettent au périmètre interne (2004) de l'élément de support à forme d'anneau unique (712, 1210, 1220, 1241, 2000) d'être apte à se déformer de manière élastique dans une direction radiale.

2. Dispositif de support de valvule (10) (30) (40) (50) (60) (110) selon la revendication 1, dans lequel l'élément de support (712, 1210, 1220, 1241, 2000) dans sa configuration déployée présente la forme d'un anneau plat annulaire, et dans lequel la différence (Rd) entre le rayon extérieur et le rayon intérieur dudit anneau annulaire se situe dans la gamme allant de 1-14 mm.

3. Dispositif de support de valvule (10) (30) (40) (50) (60) (110) selon la revendication 2, dans lequel le rapport entre Rd et l'épaisseur de l'élément de support à forme d'anneau unique (712, 1210, 1220, 1241, 2000) se situe dans la gamme allant de 10:1 et 20:1.

4. Dispositif de support de valvule (10) (30) (40) (50) (60) (110) selon la revendication 2, dans lequel le rayon intérieur de l'élément de support à forme d'anneau unique (712, 1210, 1220, 1241, 2000) se situe dans la gamme allant de 23-29 mm et le rayon extérieur de celui-ci se situe dans la gamme allant de 30 - 50 mm.

5. Dispositif de support de valvule (10) (30) (40) (50) (60) (110) selon la revendication 2, dans lequel l'épaisseur dudit dispositif de support (10) (30) (40) (50) (60) (110) se situe dans la gamme allant de 0,25 - 0,6 mm.

6. Dispositif de support de valvule (10) (30) (40) (50) (60) (110) selon la revendication 1, dans lequel les éléments stabilisant (32,36, 716) sont choisis parmi le groupe constitué de structures d'anneaux complets, d'anneaux partiels, de bras ou ailes incurvés, de bras ou ailes allongés et de bras ou ailes à levier.

7. Dispositif de support de valvule (10) (30) (40) (50) (60) (110) selon la revendication 1, comprenant en outre un élément de champ en forme d'aile formé à partir d'un tissu biocompatible (94) qui est attaché à l'élément de support à forme d'anneau unique (712, 1210, 1220, 1241, 2000).

8. Dispositif de support de valvule (10) (30) (40) (50) (60) (110) selon la revendication 1, dans lequel l'élément de support (712, 1210, 1220, 1241, 2000) est pourvu de moyens de mise en prise avec une valvule de remplacement adaptés pour venir fermement en prise avec une valvule cardiaque de remplacement.

9. Système de pose transapical ou endovasculaire pour une utilisation pour le remplacement d'une valvule mitrale (934), comprenant :
a) un dispositif de support de valvule cardiaque prothétique (10) (30) (40) (50) (60) (110) comprenant un élément de support en forme d'anneau unique (712, 1210, 1220, 1241, 2000) avec une configuration de pose affaissée et une configuration déployée, selon l'une quelconque des revendications précédentes ; et
b) une valvule cardiaque de remplacement comprenant une ancre extensible et une pluralité de clapets ; dans lequel la valvule cardiaque de remplacement est adaptée pour être fixée audit dispositif de support de valvule cardiaque prothétique (10) (30) (40) (50) (60) (110).

10. Système selon la revendication 9, dans lequel la valvule cardiaque de remplacement est une valvule aortique prothétique.
